# EUROPEAN PATENT SPECIFICATION

(11) **EP 3 875 454 B1**
(45) Date of publication and mention of the grant of the patent: **06.09.2023**
(21) Application number: 20763925.3
(22) Date of filing: 28.02.2020
(51) Int. Cl.: C07D 403/14, C07D 405/14, C07D 409/14, H10K 50/00

(54) **COMPOUND AND ORGANIC LIGHT EMITTING DEVICE COMPRISING SAME**
VERBINDUNG UND ORGANISCHE LICHTEMITTIERENDE VORRICHTUNG DAMIT
COMPOSÉ ET DISPOSITIF ÉLECTROLUMINESCENT ORGANIQUE LE COMPRENANT

(30) Priority: 28.02.2019 KR 20190024135
(43) Date of publication of application: 08.09.2021
(73) Proprietor: LG Chem, Ltd., Seoul 07336, (KR)
(72) Inventor: KIM, Minjun, Daejeon 34122 (KR); LEE, Dong Hoon, Daejeon 34122 (KR); KIM, Hyoung Seok, Daejeon 34122 (KR); LEE, Sangwoo, Daejeon 34122 (KR); SUH, Sang Duk, Daejeon 34122 (KR); KIM, Donghee, Daejeon 34122 (KR); KIM, Sunmin, Daejeon 34122 (KR); LEE, Da Jung, Daejeon 34122 (KR)
(74) Representative: Hoffmann Eitle
(86) International application number: PCT/KR2020/002906
(87) International publication number: WO 2020/175961

(56) References cited:
- WO-A1-2012/165844
- WO-A1-2015/142036
- WO-A1-2019/203550
- KR-A- 20150 108 330
- KR-A- 20150 135 109
- KR-A- 20160 022 764
- KR-A- 20180 053 121
- KR-A- 20180 125 369
- US-A1- 2015 060 833

## Description

### [Technical Field]

The present disclosure claims priority to and the benefits of Korean Patent Application No. 10-2019-0024135, filed with the Korean Intellectual Property Office on February 28, 2019.

The present specification relates to a compound, and an organic light emitting device including the same.

### [Background Art]

An organic light emission phenomenon generally refers to a phenomenon converting electrical energy to light energy using an organic material. An organic light emitting device using an organic light emission phenomenon normally has a structure including an anode, a cathode, and an organic material layer therebetween. Herein, the organic material layer is often formed in a multilayer structure formed with different materials in order to increase efficiency and stability of the organic light emitting device, and for example, may be formed with a hole injection layer, a hole transfer layer, a light emitting layer, an electron transfer layer, an electron injection layer and the like. When a voltage is applied between the two electrodes in such an organic light emitting device structure, holes and electrons are injected to the organic material layer from the anode and the cathode, respectively, and when the injected holes and electrons meet, excitons are formed, and light emits when these excitons fall back to the ground state.

Development of new materials for such an organic light emitting device has been continuously required.

### [Disclosure]

### [Technical Problem]

The present specification is directed to providing a compound, and an organic light emitting device including the same.

### [Technical Solution]

One embodiment of the present disclosure provides a compound represented by the following Chemical Formula 1. 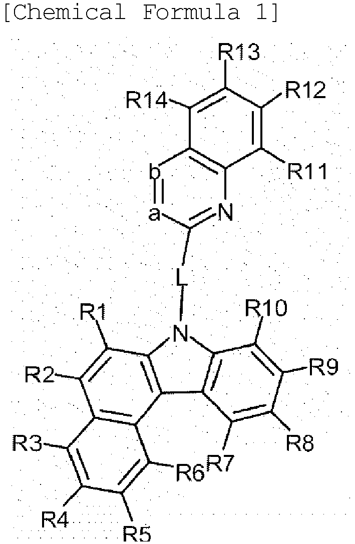
In Chemical Formula 1,
L is a direct bond, a substituted or unsubstituted arylene group, or a substituted or unsubstituted heteroarylene group,
a is CR, b is N,
R is a substituted or unsubstituted aryl group, or a substituted or unsubstituted heteroaryl group,
R1 to R6 and R11 to R14 are the same as or different from each other, and each independently hydrogen, a nitrile group, a halogen group, a substituted or unsubstituted alkyl group, a substituted or unsubstituted silyl group, a substituted or unsubstituted aryl group, or a substituted or unsubstituted heteroaryl group, and
at least one of R7 to R10 is the following Chemical Formula A, and the rest are hydrogen, 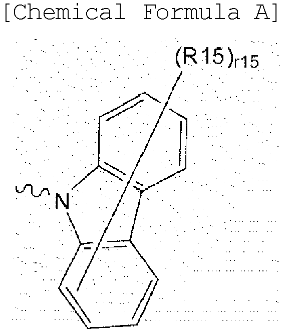
in Chemical Formula A,
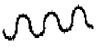 means a bond with R7 to R10,
R15 is a nitrile group, a halogen group, a substituted or unsubstituted alkyl group, a substituted or unsubstituted silyl group, a substituted or unsubstituted aryl group, or a substituted or unsubstituted heteroaryl group, or adjacent groups bond to each other to form a substituted or unsubstituted ring, and
r15 is an integer of 0 to 8, and when r15 is 2 or greater, R15s are the same as or different from each other.

Another embodiment of the present disclosure provides an organic light emitting device including a first electrode; a second electrode provided opposite to the first electrode; and one, two or more organic material layers provided between the first electrode and the second electrode, wherein one or more layers of the organic material layers include the compound.

### [Advantageous Effects]

A compound according to one embodiment of the present specification can be used as a material of an organic material layer of an organic light emitting device, and by using the same, high color purity and/or enhancement in lifetime properties can be obtained in the organic light emitting device.

### [Description of Drawings]

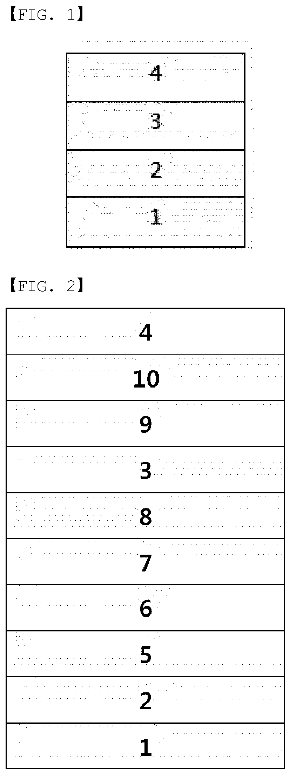

FIG. 1 and FIG. 2 illustrate an organic light emitting device according to one embodiment of the present specification.

### [Reference Numeral]

1: Substrate
2: First Electrode
3: Light Emitting Layer
4: Second Electrode
5: Hole Injection Layer
6: Hole Transfer Layer
7: Hole Transfer Auxiliary Layer
8: Electron Blocking Layer
9: Hole Blocking Layer
10: Electron Injection and Transfer Layer

### [Mode for Disclosure]

Hereinafter, the present specification will be described in more detail.

One embodiment of the present specification provides a compound represented by Chemical Formula 1.

In the compound of Chemical Formula 1, R is an aryl group or a heteroaryl group, at least one of R7 to R10 has the structure of Chemical Formula A as a substituent, and R1 to R6 and R11 to R14 do not form an additional ring.

Examples of the substituents in the present specification are described below, however, the substituents are not limited thereto.

The term "substitution" means a hydrogen atom bonding to a carbon atom of a compound being changed to another substituent. The position of substitution is not limited as long as it is a position at which the hydrogen atom is substituted, that is, a position at which a substituent can substitute, and when two or more substituents substitute, the two or more substituents may be the same as or different from each other.

In the present specification, the term "substituted or unsubstituted" means being substituted with one, two or more substituents selected from the group consisting of deuterium; a nitrile group; a substituted or unsubstituted alkyl group; a substituted or unsubstituted cycloalkyl group; a substituted or unsubstituted silyl group; a substituted or unsubstituted alkoxy group; a substituted or unsubstituted arylamine group; a substituted or unsubstituted aryl group; and a substituted or unsubstituted heterocyclic group, or being substituted with a substituent linking two or more substituents among the substituents illustrated above, or having no substituents. For example, "a
substituent linking two or more substituents" may include an aryl group substituted with an aryl group, an aryl group substituted with a heteroaryl group, a heterocyclic group substituted with an aryl group, an aryl group substituted with an alkyl group, and the like.

In the present specification, the alkyl group may be linear or branched, and although not particularly limited thereto, the number of carbon atoms is preferably from 1 to 30. Specifically, the number of carbon atoms is preferably from 1 to 20. More specifically, the number of carbon atoms is preferably from 1 to 10. Specific examples thereof may include a methyl group; an ethyl group; a propyl group; an n-propyl group; an isopropyl group; a butyl group; an n-butyl group; an isobutyl group; a tert-butyl group; a sec-butyl group; a 1-methylbutyl group; a 1-ethylbutyl group; a pentyl group; an n-pentyl group; an isopentyl group; a neopentyl group; a tert-pentyl group; a hexyl group; an n-hexyl group; a 1-methylpentyl group;; a 4-methyl-2-pentyl group; a 3,3-dimethylbutyl group; a 2-ethylbutyl group; a heptyl group; an n-heptyl group; a 1-methylhexyl group; a cyclopentylmethyl group; a cyclohexylmethyl group; an octyl group; an n-octyl group; a tert-octyl group; a 1-methylheptyl group; a 2-ethylhexyl group; a 2-propylpentyl group; an n-nonyl group; a 2,2-dimethylheptyl group; a 1-ethylpropyl group; a 1,1-dimethylpropyl group; an isohexyl group; a 2-methylpentyl group; a 4-methylhexyl group; a 5-methylhexyl group and the like, but are not limited thereto.

In the present specification, the cycloalkyl group is not particularly limited, but preferably has 3 to 30 carbon atoms, and more preferably has 3 to 20 carbon atoms. Specific examples thereof may include a cyclopropyl group; a cyclobutyl group; a cyclopentyl group; a 3-methylcyclopentyl group; a 2,3-dimethylcyclopentyl group; a cyclohexyl group; a 3-methylcyclohexyl group; a 4-methylcyclohexyl group; a 2,3-dimethylcyclohexyl group; a 3,4,5-trimethylcyclohexyl group; a 4-tert-butylcyclohexyl group; a cycloheptyl group; a cyclooctyl group and the like, but are not limited thereto.

In the present specification, the alkoxy group may be linear, branched or cyclic. The number of carbon atoms of the alkoxy group is not particularly limited, but is preferably from 1 to 30. Specifically, the number of carbon atoms is preferably from 1 to 20. More specifically, the number of carbon atoms is preferably from 1 to 10. Specific examples thereof may include a methoxy group; an ethoxy group; an n-propoxy group; an isopropoxy group; an i-propyloxy group; an n-butoxy group; an isobutoxy group; a tert-butoxy group; a sec-butoxy group; an n-pentyloxy group; a neopentyloxy group; an isopentyloxy group; an n-hexyloxy group; a 3,3-dimethylbutyloxy group; a 2-ethylbutyloxy group; an n-octyloxy group; an n-nonyloxy group; an n-decyloxy group; a benzyloxy group; a p-methylbenzyloxy group and the like, but are not limited thereto.

In the present specification, the amine group may be selected from the group consisting of -NH₂; an alkylamine group; an N-alkylarylamine group; an arylamine group; an N-arylheteroarylamine group; an N-alkylheteroarylamine group and a heteroarylamine group, and although not particularly limited thereto, the number of carbon atoms is preferably from 1 to 30. Specific examples of the amine
group may include a methylamine group; a dimethylamine group; an ethylamine group; a diethylamine group; a phenylamine group; a naphthylamine group; a biphenylamine group; an anthracenylamine group; a 9-methylanthracenylamine group; a diphenylamine group; an N-phenylnaphthylamine group; a ditolylamine group; an N-phenyltolylamine group; a triphenylamine group; an N-phenylbiphenylamine group; an N-biphenylnaphthylamine group; an N-naphthylfluorenylamine group; an N-phenylphenanthrenylamine group; an N-biphenylphenanthrenylamine group; an N-phenylfluorenylamine group; an N-phenylterphenylamine group; an N-phenanthrenylfluorenylamine group; an N-biphenylfluorenylamine group and the like, but are not limited thereto.

In the present specification, the silyl group may be represented by a chemical formula of -SiRaRbRc, and Ra, Rb and Rc are the same as or different from each other, and may be each independently hydrogen; a substituted or unsubstituted alkyl group; or a substituted or unsubstituted aryl group. Specific examples of the silyl group may include a trimethylsilyl group; a triethylsilyl group; a t-butyldimethylsilyl group; a vinyldimethylsilyl group; a propyldimethylsilyl group; a triphenylsilyl group; a diphenylsilyl group; a phenylsilyl group and the like, but are not limited thereto.

In the present specification, the aryl group is not particularly limited, but preferably has 6 to 30 carbon atoms, and more preferably has 6 to 20 carbon atoms. The aryl group may be monocyclic or polycyclic. When the aryl group is a monocyclic aryl group, the number of carbon atoms is not particularly limited, but
is preferably from 6 to 30. More specifically, the number of carbon atoms is preferably from 6 to 20. Specific examples of the monocyclic aryl group may include a phenyl group; a biphenyl group; a terphenyl group and the like, but are not limited thereto. When the aryl group is a polycyclic aryl group, the number of carbon atoms is not particularly limited, but is preferably from 10 to 30. More specifically, the number of carbon atoms is preferably from 10 to 20. Specific examples of the polycyclic aryl group may include a naphthyl group; an anthracenyl group; a phenanthryl group; a triphenylene group; a pyrenyl group; a phenalenyl group; a perylenyl group; a chrysenyl group; a fluorenyl group and the like, but are not limited thereto.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present specification, examples of the arylamine group include a substituted or unsubstituted monoarylamine group, a substituted or unsubstituted diarylamine group, or a substituted or unsubstituted triarylamine group. The aryl group in the arylamine group may be a monocyclic aryl group or a polycyclic aryl group. The arylamine group including two or more aryl groups may include monocyclic aryl groups, polycyclic aryl groups, or both monocyclic aryl groups and polycyclic aryl groups. For example, the aryl group in the arylamine group may be selected from among the examples of the aryl group described above.

In the present specification, the heteroaryl group is a group including one or more atoms that are not carbon, that is, heteroatoms, and specifically, the heteroatom may include one or more atoms selected from the group consisting of O, N, Se, S and the like. The number of carbon atoms is not particularly limited, but is preferably from 2 to 30 and more preferably from 2 to 20, and the heteroaryl group may be monocyclic or polycyclic. Examples of the heteroaryl group may include a thiophene group; a furanyl group; a pyrrole group; an imidazolyl group; a thiazolyl group; an oxazolyl group; an oxadiazolyl group; a pyridyl group; a bipyridyl group; a pyrimidyl group; a triazinyl group; a triazolyl group; an acridyl group; a pyridazinyl group; a pyrazinyl group; a quinolinyl group; a quinazolinyl group; a quinoxalinyl group; a phthalazinyl group; a pyridopyrimidyl group; a pyridopyrazinyl group; a pyrazinopyrazinyl group; an isoquinolinyl group; an indolyl group; a carbazolyl group; a benzoxazolyl group; a benzimidazolyl group; a benzothiazolyl group; a benzocarbazolyl group; a benzothiophene group; a dibenzothiophene group; a benzofuranyl group; a phenanthrolinyl group; an isoxazolyl group; a thiadiazolyl group; a phenothiazinyl group; a dibenzofuranyl group and the like, but are not limited thereto.

In the present specification, the arylene group has the same definition as the aryl group except for being divalent.

In the present specification, the heteroarylene group has the same definition as the heteroaryl group except for being divalent.

In the present specification, when adjacent substituents bonding to each other to form a ring, the ring may be a hydrocarbon ring, an aromatic ring or a heteroring. Herein, the hydrocarbon ring has the same definition as the cycloalkyl group except for those that are not monovalent, the aromatic ring has the same definition as the aryl group except for those that are not monovalent, and the heteroring has the same definition as the heteroaryl group except for those that are not monovalent.

In one embodiment of the present specification, R1 to R6 and R11 to R14 are hydrogen.

In one embodiment of the present specification, r15 is 0.

In one embodiment of the present specification, R15 is a nitrile group, a halogen group, an alkyl group, an aryl group or a heteroaryl group, or adjacent groups bond to each other to form a substituted or unsubstituted ring.

In one embodiment of the present specification, R15 is an aryl group having 6 to 30 carbon atoms or a heteroaryl group having 3 to 30 carbon atoms, or adjacent groups bond to each other to form a substituted or unsubstituted aromatic ring, or a substituted or unsubstituted heteroring.

In one embodiment of the present specification, adjacent groups R15 bond to each other to form an aromatic ring or a heteroring.

In one embodiment of the present specification, adjacent groups R15 bond to each other to form an aromatic ring.

In one embodiment of the present specification, , adjacent groups R15 bond to each other to form a benzene ring.

In one embodiment of the present specification, one of R7 to R10 is Chemical Formula A, and the rest are hydrogen.

In one embodiment of the present specification, R7 of R7 to R10 is Chemical Formula A, and the rest are hydrogen.

In one embodiment of the present specification, R8 of R7 to R10 is Chemical Formula A, and the rest are hydrogen.

In one embodiment of the present specification, R9 of R7 to R10 is Chemical Formula A, and the rest are hydrogen.

In one embodiment of the present specification, R10 of R7 to R10 is Chemical Formula A, and the rest are hydrogen.

In one embodiment of the present specification, R is a substituted or unsubstituted aryl group.

In one embodiment of the present specification, R is a substituted or unsubstituted heteroaryl group.

In one embodiment of the present specification, R is an aryl group having 6 to 30 carbon atoms unsubstituted or substituted with an aryl group; an aryl group having 6 to 30 carbon atoms substituted with an alkyl group; or a heteroaryl group having 3 to 30 carbon atoms unsubstituted or substituted with an aryl group.

In one embodiment of the present specification, R is an aryl group having 6 to 30 carbon atoms unsubstituted or substituted with an aryl group having 6 to 20 carbon atoms; an aryl group having 6 to 30 carbon atoms substituted with an alkyl group having 1 to 10 carbon atoms; or a heteroaryl group having 3 to 30 carbon atoms unsubstituted or substituted with an aryl group having 6 to 20 carbon atoms.

In one embodiment of the present specification, R is an aryl group having 6 to 30 carbon atoms unsubstituted or substituted with an aryl group having 6 to 20 carbon atoms.

In one embodiment of the present specification, R is an aryl group having 6 to 30 carbon atoms unsubstituted or substituted with an alkyl group having 1 to 10 carbon atoms.

In one embodiment of the present specification, R is a substituted or unsubstituted heteroaryl group having 3 to 30 carbon atoms.

In one embodiment of the present specification, R is an aryl group having 6 to 30 carbon atoms unsubstituted or substituted with a methyl group, an ethyl group, a propyl group, an isopropyl group, a butyl group or a tert-butyl group.

In one embodiment of the present specification, R is an aryl group having 6 to 30 carbon atoms unsubstituted or substituted with any one or more selected from among a phenyl group, a biphenyl group, a naphthyl group, an anthracene group, a phenanthrene group, a terphenyl group and a triphenyl group.

In one embodiment of the present specification, R is a phenyl group, a naphthyl group, a biphenyl group, a terphenyl group, an anthracene group, a phenanthrene group, a fluorene group, a triphenylene group or a pyrene group, and
the phenyl group, the naphthyl group, the biphenyl group, the terphenyl group, the anthracene group, the phenanthrene group, the fluorene group, the triphenylene group or the pyrene group is unsubstituted or substituted with any one or more selected from among a methyl group, an ethyl group, a propyl group, an isopropyl group, a butyl group, a tert-butyl group, a phenyl group, a biphenyl group, a naphthyl group, an anthracene group, a phenanthrene group, a terphenyl group and a triphenyl group.

In one embodiment of the present specification, R is a phenyl group, a biphenyl group, a terphenyl group, a naphthyl group, a phenanthrene group or a fluorene group, and
the phenyl group, the biphenyl group, the terphenyl group, the naphthyl group, the phenanthrene group or the fluorene group is unsubstituted or substituted with a phenyl group, a naphthyl group or a methyl group.

In one embodiment of the present specification, R is a phenyl group; a phenyl group substituted with a naphthyl group; a naphthyl group; a naphthyl group substituted with a phenyl group; a biphenyl group; a terphenyl group; a phenanthrene group; or a fluorene group substituted with a methyl group.

In one embodiment of the present specification, R is a heteroaryl group having 3 to 30 carbon atoms and including any one or more of N, O and S unsubstituted or substituted with an aryl group.

In one embodiment of the present specification, R is a heteroaryl group having 3 to 30 carbon atoms and including any one or more of N, O and S unsubstituted or substituted with an aryl group having 6 to 30 carbon atoms.

In one embodiment of the present specification, R is a pyridine group, a pyrimidine group, a triazine group, a dibenzofuran group, a dibenzothiophene group or a carbazole group, and
the pyridine group, the pyrimidine group, the triazine group, the dibenzofuran group, the dibenzothiophene group or the carbazole group is unsubstituted or substituted with an aryl group having 6 to 30 carbon atoms.

In one embodiment of the present specification, R is a pyridine group, a pyrimidine group, a triazine group, a dibenzofuran group, a dibenzothiophene group or a carbazole group, and
the pyridine group, the pyrimidine group, the triazine group, the dibenzofuran group, the dibenzothiophene group or the carbazole group is unsubstituted or substituted with a phenyl group, a biphenyl group, a terphenyl group, an anthracene group, a phenanthrene group or a naphthyl group.

In one embodiment of the present specification, R is a dibenzofuran group, a dibenzothiophene group, or a carbazole group unsubstituted or substituted with a phenyl group.

In one embodiment of the present specification, R is a phenyl group; a phenyl group substituted with a naphthyl group; a naphthyl group; a naphthyl group substituted with a phenyl group; a biphenyl group; a terphenyl group; a phenanthrene group; a fluorene group substituted with a methyl group, a dibenzofuran group, a dibenzothiophene group, or a carbazole group unsubstituted or substituted with a phenyl group.

In one embodiment of the present specification, L is a direct bond, a substituted or unsubstituted arylene group having 6 to 30 carbon atoms, or a substituted or unsubstituted heteroarylene group having 3 to 30 carbon atoms.

In one embodiment of the present specification, L is a direct bond, or an arylene group having 6 to 30 carbon atoms.

In one embodiment of the present specification, L is a direct bond, or an arylene group having 6 to 20 carbon atoms.

In one embodiment of the present specification, L is a direct bond, or an arylene group having 6 to 12 carbon atoms.

In one embodiment of the present specification, L is a direct bond, a phenylene group, a divalent biphenyl group or a divalent naphthyl group.

In one embodiment of the present specification, L is a direct bond.

In one embodiment of the present specification, Chemical Formula 1 is any one selected from among the following compounds.

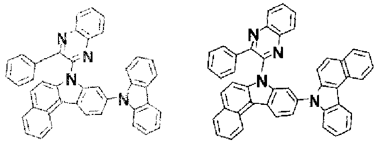

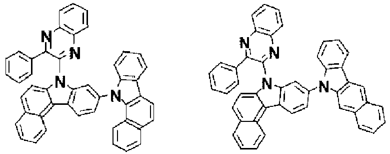

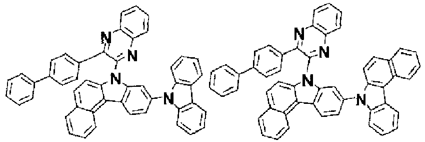

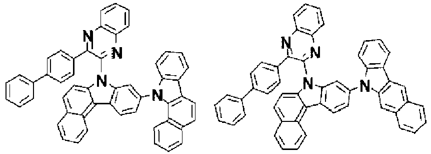

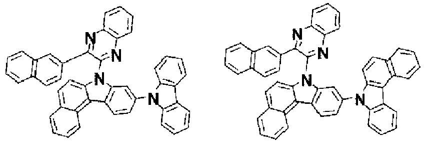

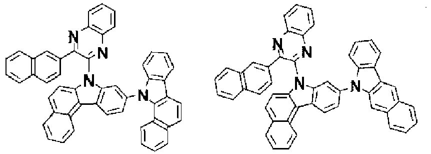

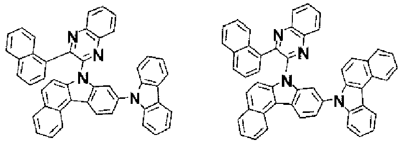

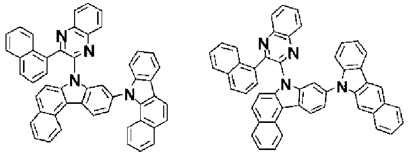

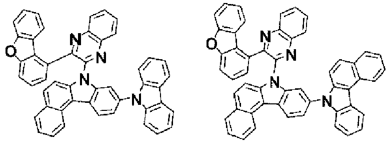

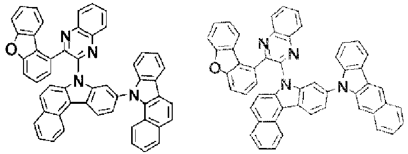

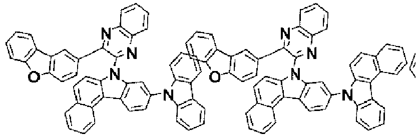

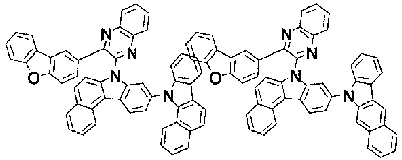

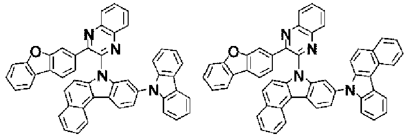

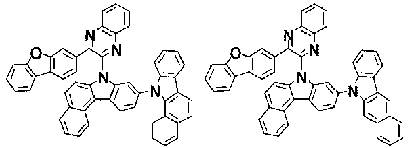

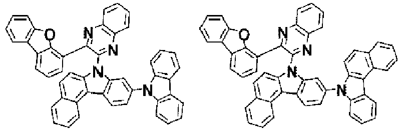

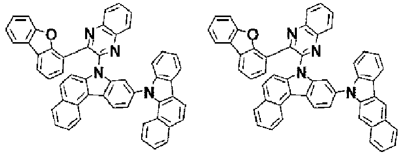

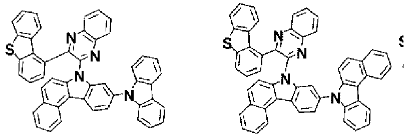

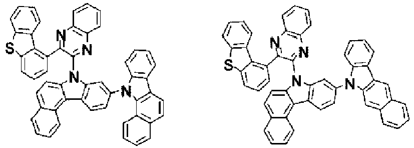

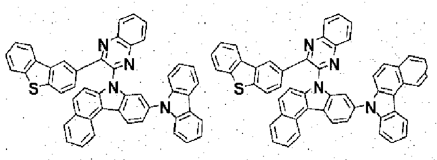

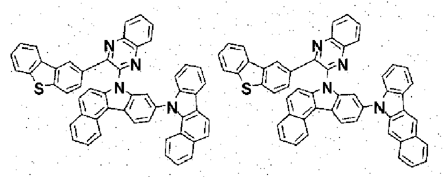

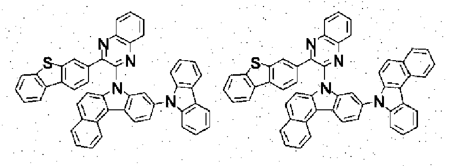

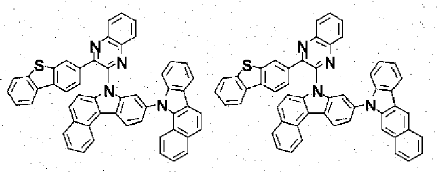

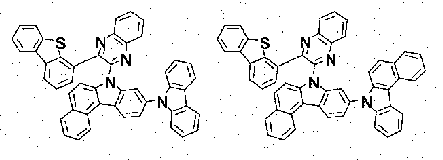

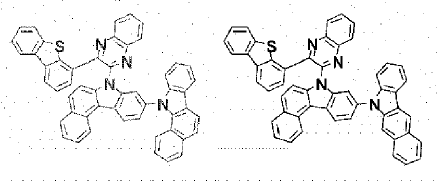

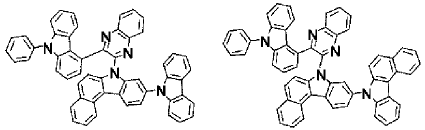

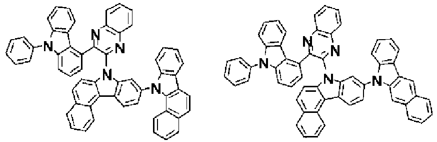

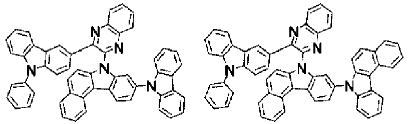

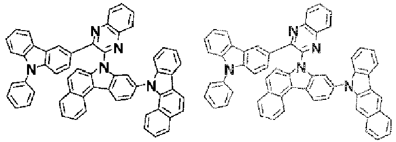

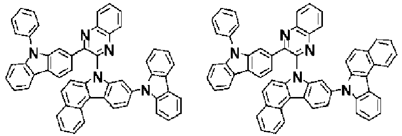

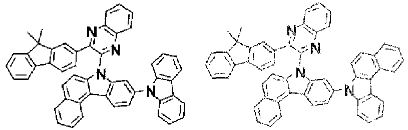

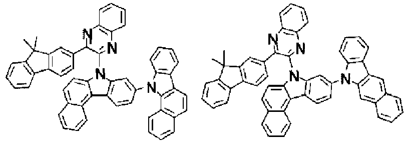

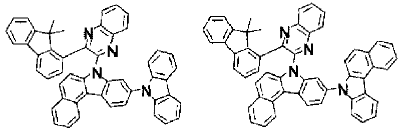

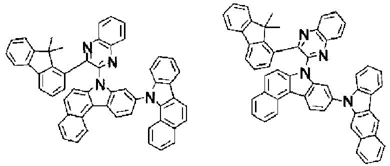

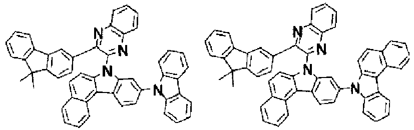

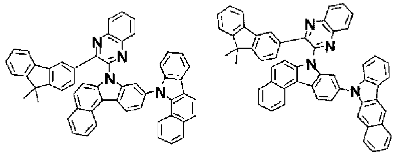

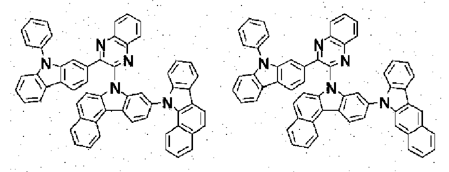

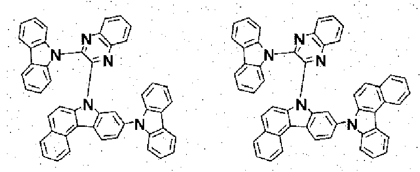

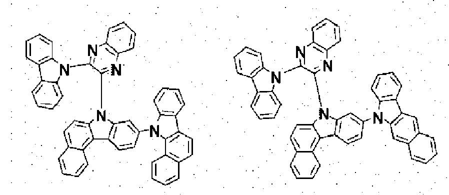

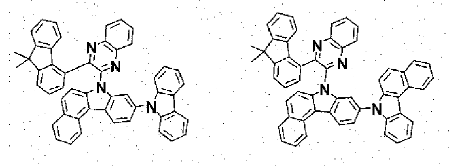

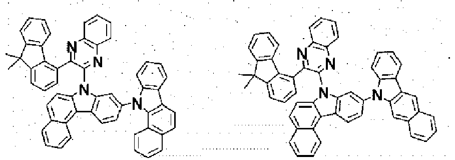

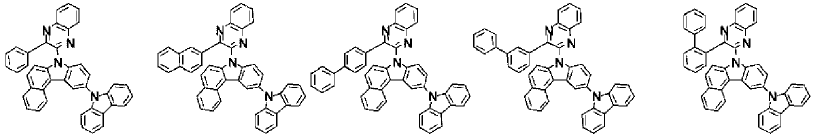

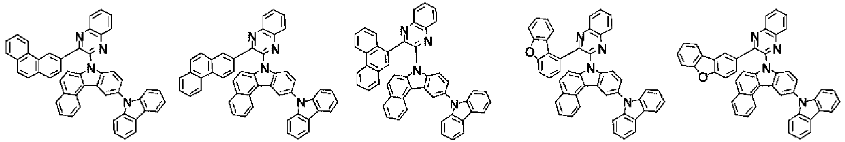

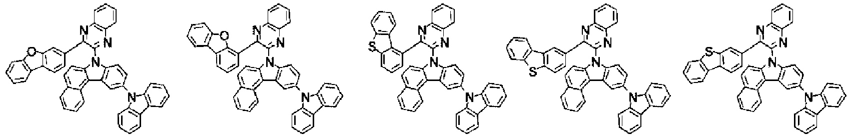

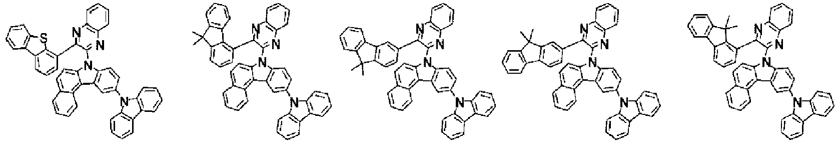

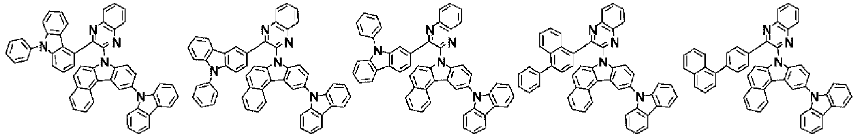

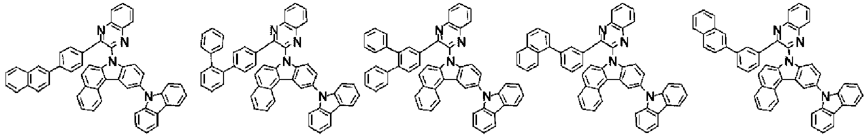

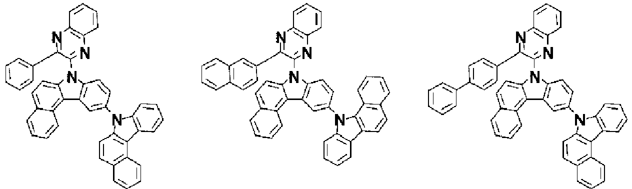

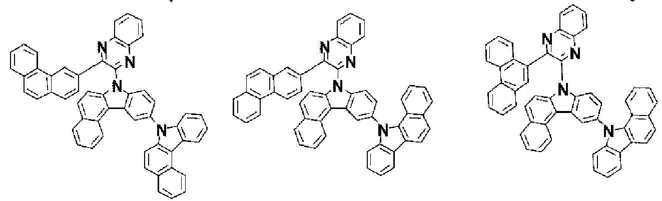

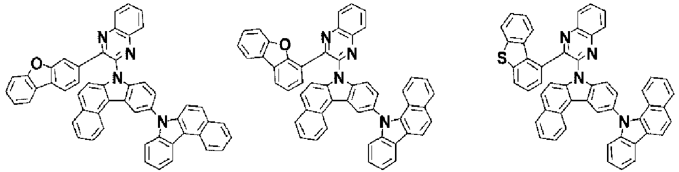

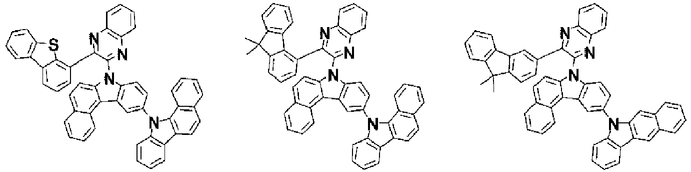

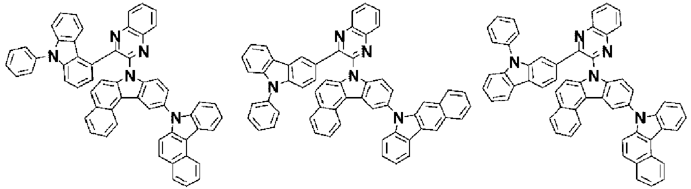

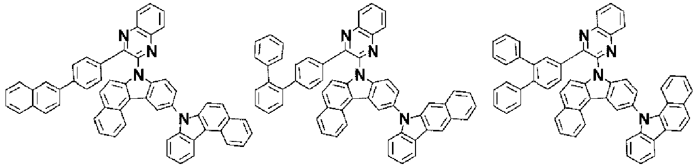

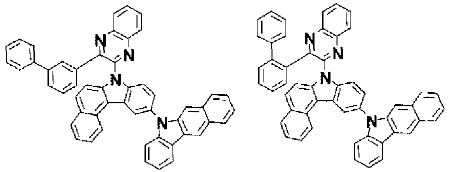

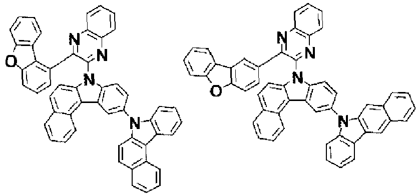

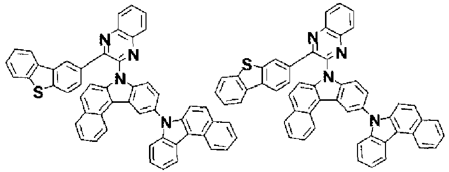

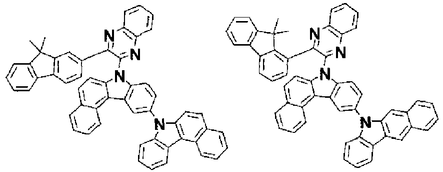

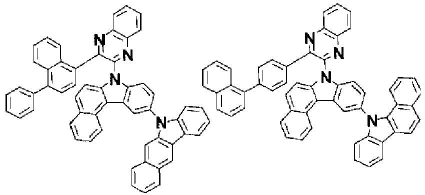

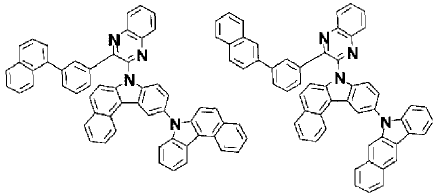

In the present specification, a description of a certain part "including" certain constituents means capable of further including other constituents, and does not exclude other constituents unless particularly stated on the contrary.

In the present specification, a description of a certain member being placed "on" another member includes not only a case of the one member in contact with the another member but a case of still another member being present between the two members.

An organic light emitting device of the present disclosure includes a first electrode; a second electrode provided opposite to the first electrode; and one, two or more organic material layers provided between the first electrode and the second electrode, wherein one or more layers of the organic material layers may include the compound described above.

For example, the organic light emitting device of the present disclosure may have structures as illustrated in FIG. 1 and FIG. 2, however, the structure is not limited thereto.

FIG. 1 illustrates a structure of the organic light emitting device in which a first electrode (2), a light emitting layer (3) and a second electrode (4) are consecutively laminated on a substrate (1).

FIG. 1 illustrates the organic light emitting device, and the structure is not limited thereto.

FIG. 2 illustrates a structure of the organic light emitting device in which a first electrode (2), a hole injection layer (5), a hole transfer layer (6), a hole transfer auxiliary layer (7), an electron blocking layer (8), a light emitting layer (3), a hole blocking layer (9), an electron injection and transfer layer (10) and a second electrode (4) are consecutively laminated on a substrate (1). The compound of Chemical Formula 1 may be preferably included in the light emitting layer (3), however, the structure is not limited thereto.

Additional layers may be further included in addition to the laminated structures used in FIG. 1 and FIG. 2, or some layers may be removed therefrom when used.

In one embodiment of the present disclosure, the first electrode is an anode, and the second electrode is a cathode.

In one embodiment of the present disclosure, the first electrode is a cathode, and the second electrode is an anode.

In one embodiment of the present disclosure, the organic material layer includes a light emitting layer, and the light emitting layer includes the compound.

In one embodiment of the present disclosure, the organic material layer includes a light emitting layer, and the light emitting layer includes a host and a dopant.

In one embodiment of the present disclosure, the organic material layer includes a light emitting layer, and the light emitting layer includes a host and a dopant in a mass ratio of 99:1 to 70:30.

In one embodiment of the present disclosure, the light emitting layer includes the compound of Chemical Formula 1 as a host.

In one embodiment of the present disclosure, the light emitting layer includes the compound of Chemical Formula 1 as a red host.

In one embodiment of the present disclosure, the light emitting layer further includes a dopant.

In one embodiment of the present disclosure, the light emitting layer includes a metal complex compound as a dopant material.

In one embodiment of the present disclosure, the light emitting layer includes an iridium-based complex compound as a dopant material.

In one embodiment of the present disclosure, the light emitting layer may use compounds selected from among the following structural formulae as a dopant material, however, the dopant material is not limited thereto.

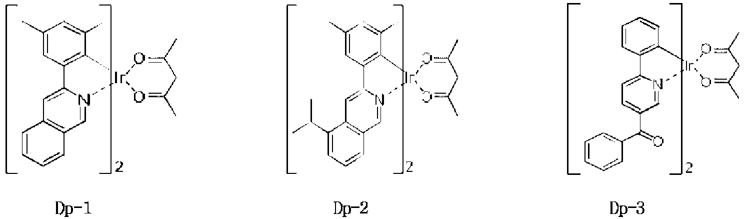

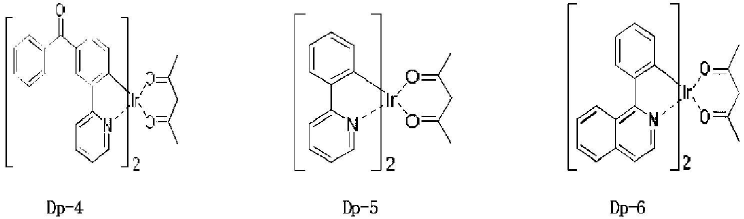

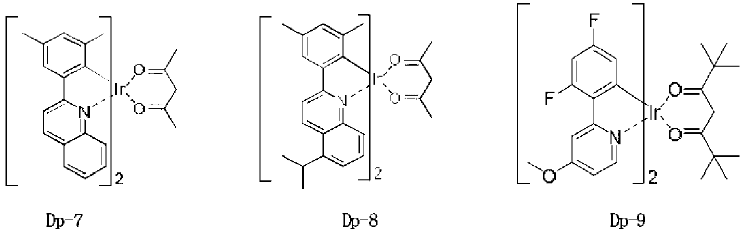

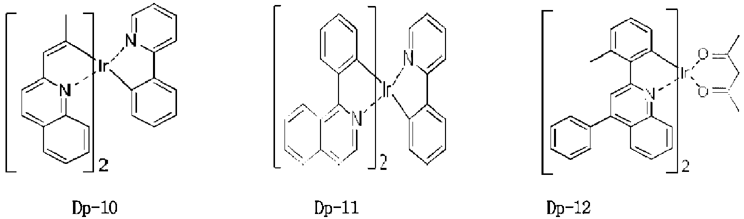

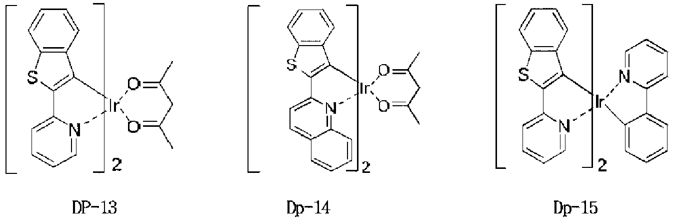

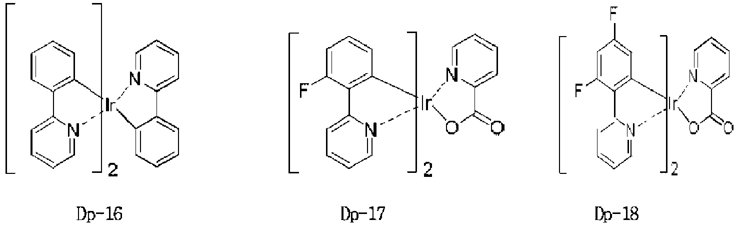

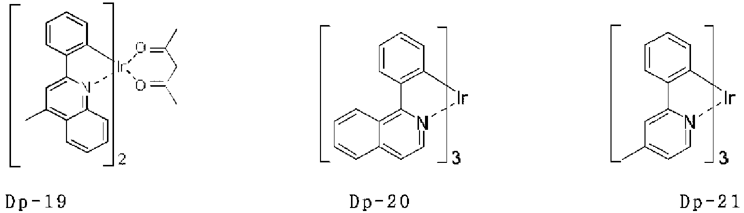

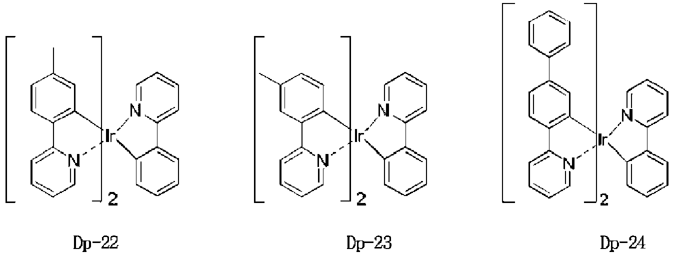

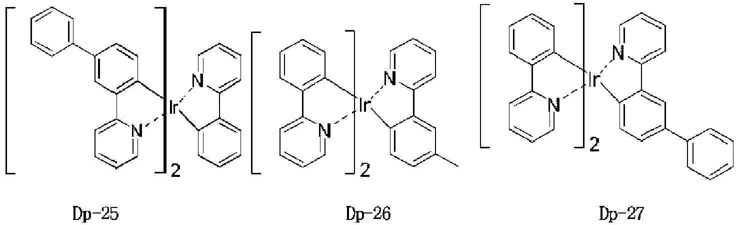

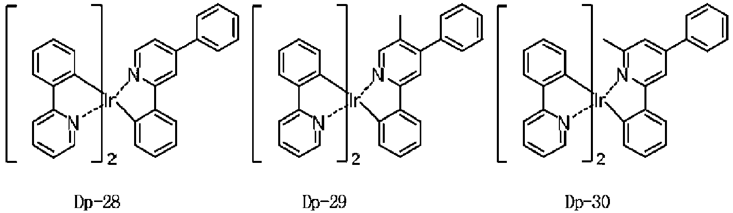

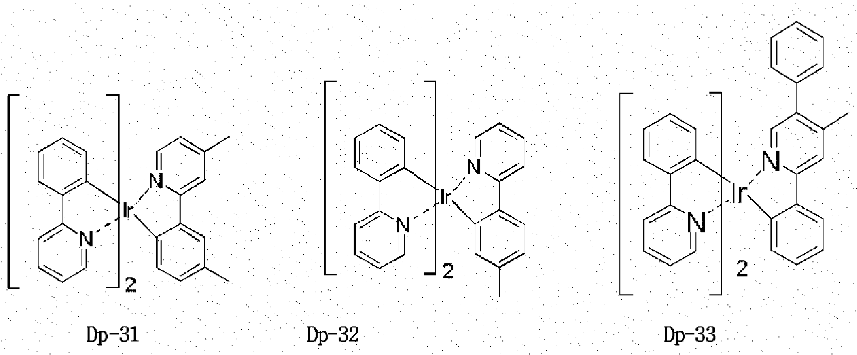

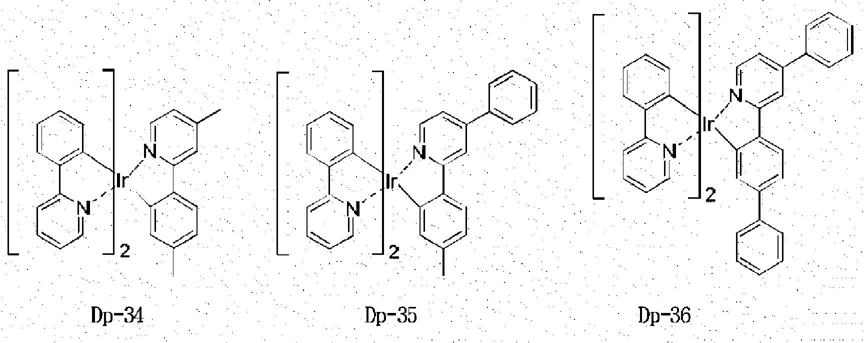

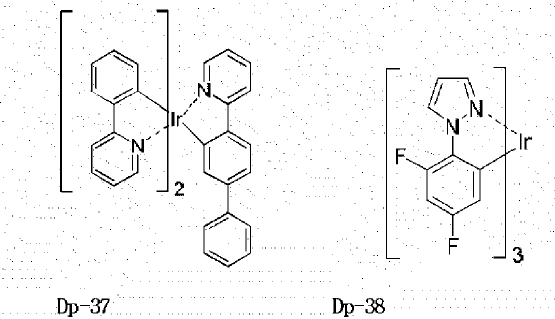

In one embodiment of the present disclosure, the organic material layer includes a hole injection layer, a hole transfer layer, or a hole injection and transfer layer, and the hole injection layer, the hole transfer layer, or the hole injection and transfer layer may include the compound of Chemical Formula 1.

In one embodiment of the present disclosure, the organic material layer includes an electron injection layer, an electron transfer layer, or an electron injection and transfer layer, and the electron injection layer, the electron transfer layer, or the electron injection and transfer layer may include the compound of Chemical Formula 1.

In one embodiment of the present disclosure, the organic material layer includes an electron blocking layer or a hole blocking layer, and the electron blocking layer or the hole blocking layer may include the compound of Chemical Formula 1.

For example, the organic light emitting device according to the present disclosure may be manufactured by forming an anode on a substrate by depositing a metal, a metal oxide having conductivity, or an alloy thereof using a physical vapor deposition (PVD) method such as sputtering or e-beam evaporation, forming an organic material layer including a hole injection layer, a hole transfer layer, a light emitting layer and an electron transfer layer, and an organic material layer including the compound of Chemical Formula 1 thereon, and then depositing a material capable of being used as a cathode thereon. In addition to such a method, the organic light emitting device may also be manufactured by consecutively depositing a cathode material, an organic material layer and an anode material on a substrate.

As the anode material, materials having large work function are normally preferred so that hole injection to an organic material layer is smooth. Specific examples of the anode material capable of being used in the present disclosure include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDT), polypyrrole and polyaniline, but are not limited thereto.

As the cathode material, materials having small work function are normally preferred so that electron injection to an organic material layer is smooth. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

The hole injection material is a material that may favorably receive holes from an anode at a low voltage, and the highest occupied molecular orbital (HOMO) of the hole injection material is preferably in between the work function of an anode material and the HOMO of surrounding organic material layers. Specific examples of the hole injection material include metal porphyrins, oligothiophene, arylamine-based organic materials, hexanitrile hexaazatriphenylene-based organic materials, quinacridone-based organic materials, perylene-based organic materials, anthraquinone, and polyaniline- and polythiophene-based conductive polymers, and the like, but are not limited thereto.

As the hole transfer material, materials capable of receiving holes from an anode or a hole injection layer, moving the holes to a light emitting layer, and having high mobility for the holes are suited. Specific examples thereof include arylamine-based organic materials, conductive polymers, block copolymers having conjugated parts and non-conjugated parts together, and the like, but are not limited thereto.

The light emitting material is a material capable of emitting light in a visible light region by receiving holes and electrons from a hole transfer layer and an electron transfer layer, respectively, and binding the holes and the electrons, and is preferably a material having favorable quantum efficiency for fluorescence or phosphorescence. Specific examples thereof include 8-hydroxy-quinoline aluminum complexes (Alq₃); carbazole-based compounds; dimerized styryl compounds; BAlq; 10-hydroxybenzoquinoline-metal compounds; benzoxazole, benzothiazole and benzimidazole-based compounds; poly(p-phenylenevinylene) (PPV)-based polymers; spiro compounds; polyfluorene; rubrene, and the like, but are not limited thereto.

When the organic light emitting device includes a plurality of organic material layers, the organic material layers may be formed with the same materials or different materials.

The organic light emitting device of the present specification may be manufactured using materials and methods known in the art except that one or more layers of the organic material layers are formed using the compound.

Another embodiment of the present specification provides a method for manufacturing an organic light emitting device formed using the compound.

The dopant material may include aromatic compounds, styrylamine compounds, boron complexes, fluoranthene compounds, metal complexes and the like. Specifically, the aromatic compound is a fused aromatic ring derivative having a substituted or unsubstituted arylamino group, and arylamino group-including pyrene, anthracene, chrysene, peryflanthene and the like may be included. As the styrylamine compound, compounds in which substituted or unsubstituted arylamine is substituted with at least one arylvinyl group may be used, and one, two or more substituents selected from the group consisting of an aryl group, a silyl group, an alkyl group, a cycloalkyl group and an arylamino group substitute or unsubstitute. Specifically, styrylamine, styryldiamine, styryltriamine, styryltetraamine and the like may be included, however, the styrylamine compound is not limited thereto. In addition, as the metal complex, iridium complexes, platinum complexes and the like may be included, however, the metal complex is not limited thereto.

The electron transfer layer is a layer receiving electrons from an electron injection layer and transferring the electrons to a light emitting layer, and as the electron transfer material, materials capable of favorably receiving electrons from a cathode, moving the electrons to a light emitting layer, and having high mobility for the electrons are suited. Specific examples thereof include Al complexes of 8-hydroxyquinoline; complexes including Alq₃; organic radical compounds; hydroxyflavon-metal complexes, and the like, but are not limited thereto. The electron transfer layer may be used together with any desired cathode material as used in the art. Particularly, examples of the suitable cathode material may include common materials having low work function and having an aluminum layer or a silver layer following. Specifically, cesium, barium, calcium, ytterbium and samarium are included, and in each case, an aluminum layer or a silver layer follows.

The electron injection layer is a layer injecting electrons from an electrode, and compounds having an electron transferring ability, having an electron injection effect from a cathode, having an excellent electron injection effect for a light emitting layer or light emitting material, and preventing excitons generated in the light emitting layer from moving to a hole injection layer, and in addition thereto, having an excellent thin film forming ability are preferred. Specific examples thereof include fluorenone, anthraquinodimethane, diphenoquinone, thiopyran dioxide, oxazole, oxadiazole, triazole, imidazole, perylene tetracarboxylic acid, fluorenylidene methane, anthrone or the like, and derivatives thereof, metal complex compounds, nitrogen-containing 5-membered ring derivatives, and the like, but are not limited thereto.

The metal complex compound includes 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato) (1-naphtholato)aluminum, bis(2-methyl-8-quinolinato) (2-naphtholato)gallium and the like, but is not limited thereto.

The hole blocking layer is a layer blocking holes from reaching a cathode, and may be generally formed under the same condition as the hole injection layer. Specific examples thereof may include oxadiazole derivatives, triazole derivatives, phenanthroline derivatives, BCP, aluminum complexes and the like, but are not limited thereto.

The organic light emitting device according to the present specification may be a top-emission type, a bottom-emission type or a dual-emission type depending on the materials used.

The organic light emitting device of the present disclosure may be manufactured using common organic light emitting device manufacturing methods and materials except that one or more organic material layers are formed using the compound described above.

Preparation of the compounds of the present disclosure and manufacturing of the organic light emitting device using the same will be described below. These are for helping those skilled in the art understand the present disclosure, and the present disclosure is not limited thereto. All the compounds described in the present disclosure may be prepared by varying types of starting materials, substituents and positions of substitutions.

### Synthesis Example and Example

Compounds of the present disclosure were prepared using a Buchwald-Hartwig coupling reaction, a Heck coupling reaction, a Suzuki coupling reaction or the like as a representative reaction.

The compounds of Synthesis Examples 1, 3, 4, 6-12 form part of the invention, whereas the compounds of Synthesis Examples 2, 5, 13-18 do not form part of the invention.

### Preparation Example 1.

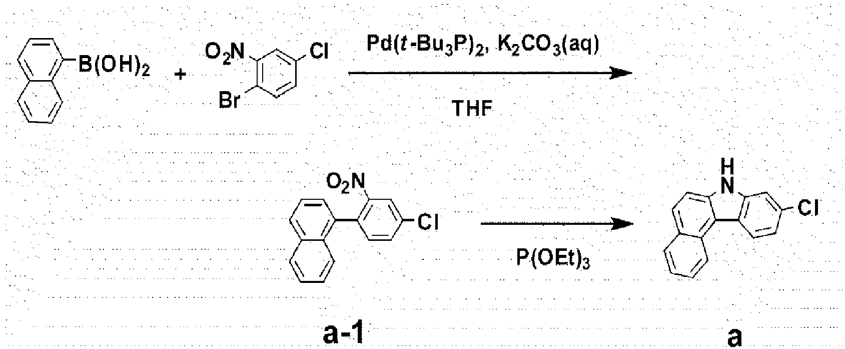

### 1) Preparation of Chemical Formula a-1

Naphthalen-1-ylboronic acid (100 g, 581.2 mmol) and 1-bromo-4-chloro-2-nitrobenzene (150.2 g, 639.3 mmol) were introduced to THF (2000 ml) under the nitrogen atmosphere, and the result was stirred and refluxed. After that, potassium carbonate (321.3 g, 2324.6 mmol) dissolved in water (964 ml) was introduced thereto, and after sufficiently stirring the result, bis(tri-tert-butylphosphine)palladium(0) (3 g, 5.8 mmol) was introduced thereto. After reacting for 2 hours, the result was cooled to room temperature, and after separating the organic layer and the water layer, the organic layer was distilled. This was dissolved in chloroform again, washed twice with water, and then the organic layer was separated, stirred after introducing anhydrous magnesium sulfate thereto and filtered, and the filtrate was vacuum distilled. The concentrated compound was purified using silica gel column chromatography to prepare Chemical Formula a-1 (111.9 g).
(Yield 68%, MS: [M+H]⁺=284)

### 2) Preparation of Chemical Formula a

Chemical Formula a-1 (111.9 g, 1.0 eq.) was introduced to P(OEt)₃ (500 mL), and the result was stirred under reflux. After 3 hours, the reaction material was poured into water to drop crystals, and filtered. The filtered solids were completely dissolved in chloroform, and then washed with water. The solution in which a product was dissolved was vacuum concentrated to drop crystals, and cooled and then filtered. The result was purified using column chromatography to obtain Chemical Formula a (50.6 g).
(Yield 51%, [M+H]⁺=252)

### Preparation Example 2.

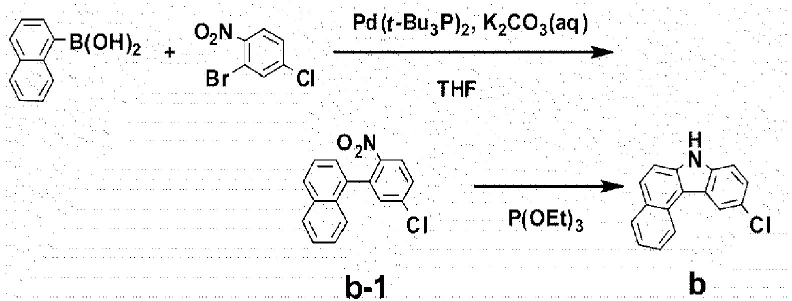

Chemical Formula b-1 was synthesized using 2-bromo-4-chloro-1-nitrobenzene instead of 1-bromo-4-chloro-2-nitrobenzene used in Preparation Example 1, and Chemical Formula b was subsequently synthesized.

### Synthesis Example 1

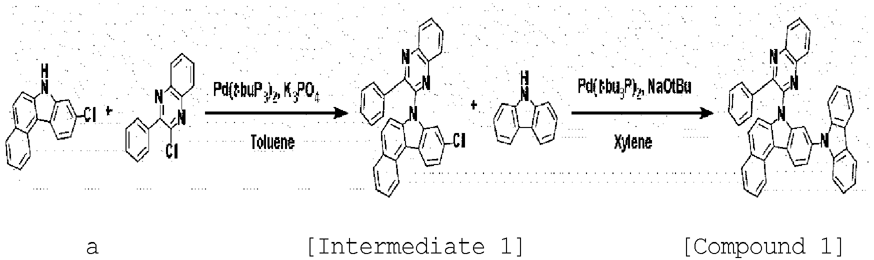

### Synthesis of Intermediate 1

Chemical Formula a (20 g, 79.7 mmol), 2-chloro-3-phenylquinoxaline (19.1 g, 79.7 mmol) and tripotassium phosphate (15.3 g, 159.3 mmol) were introduced to toluene (400 ml) under the nitrogen atmosphere, and the result was stirred and refluxed. After that, bis(tri-tert-butylphosphine)palladium(0) (0.8 g, 1.6 mmol) was introduced thereto. The reaction was terminated after 2 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Intermediate 1 (21.8 g). (Yield 60%, MS: [M+H]⁺=456)

### Synthesis of Compound 1

Intermediate 1 (20 g, 43.9 mmol), 9H-carbazole (7.3 g, 43.9 mmol) and sodium tert-butoxide (8.4 g, 87.9 mmol) were introduced to xylene (400 ml) under the nitrogen atmosphere, and the result was stirred and refluxed. After that, bis(tri-tert-butylphosphine)palladium(0) (0.4 g, 0.9 mmol) was introduced thereto. The reaction was terminated after 2 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 1 (15.2 g). (Yield 59%, MS: [M+H]+=587)

In the following Synthesis Examples 2 to 18, Intermediates 2 to 18 were prepared by varying starting materials and substituents in Synthesis of Intermediate 1.

### Synthesis Example 2

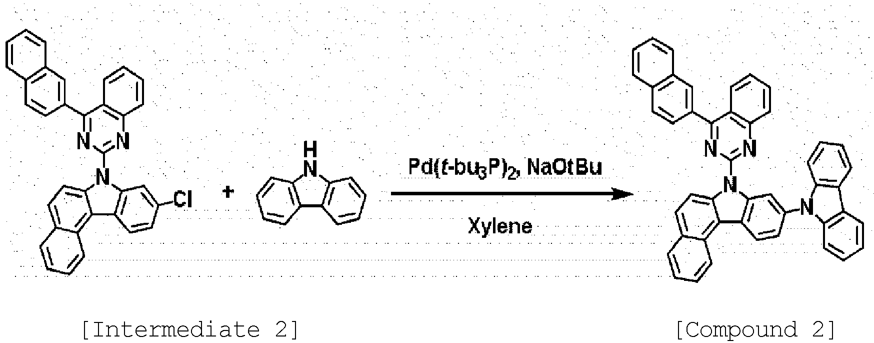

Intermediate 2 (10 g, 19.8 mmol), 9H-carbazole (3.5 g, 20.8 mmol) and sodium tert-butoxide (3.8 g, 39.6 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 2 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 2 (8.2 g). (Yield 65%, MS: [M+H]⁺=637)

### Synthesis Example 3

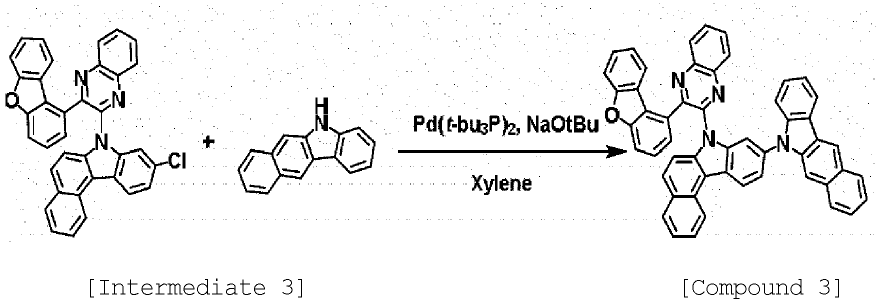

Intermediate 3 (10 g, 18.3 mmol), 5H-benzo[b]carbazole (4.2 g, 19.3 mmol) and sodium tert-butoxide (3.5 g, 36.7 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 2 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 3 (7.3 g). (Yield 55%, MS: [M+H]⁺=727)

### Synthesis Example 4

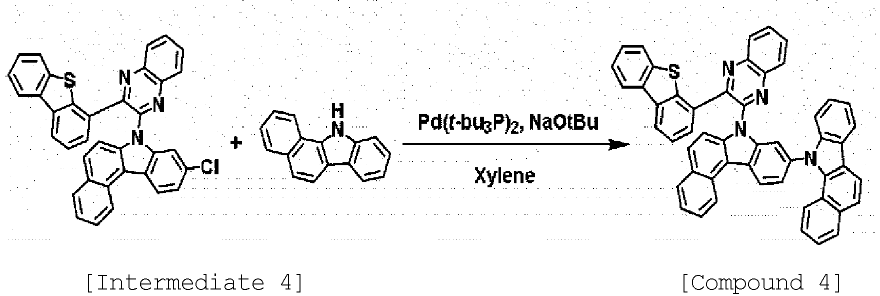

Intermediate 4 (10 g, 17.8 mmol), 11H-benzo[a]carbazole (4.1 g, 18.7 mmol) and sodium tert-butoxide (3.4 g, 35.6 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 4 (8.9 g). (Yield 67%, MS: [M+H]⁺=743)

### Synthesis Example 5

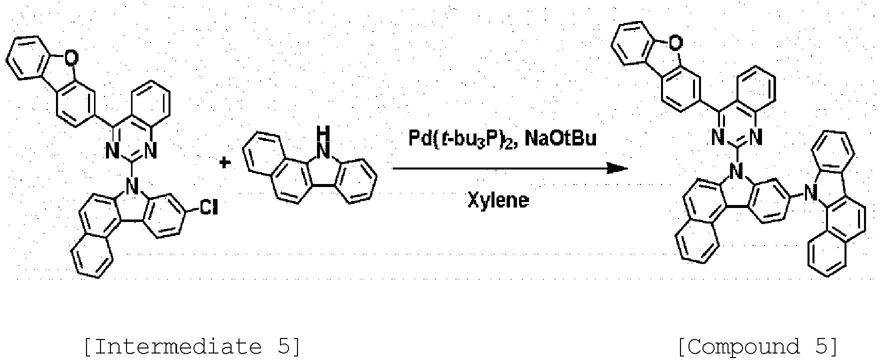

Intermediate 5 (10 g, 18.3 mmol), 11H-benzo[a]carbazole (4.2 g, 19.3 mmol) and sodium tert-butoxide (3.5 g, 36.7 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 5 (9.3 g). (Yield 70%, MS: [M+H]⁺=727)

### Synthesis Example 6

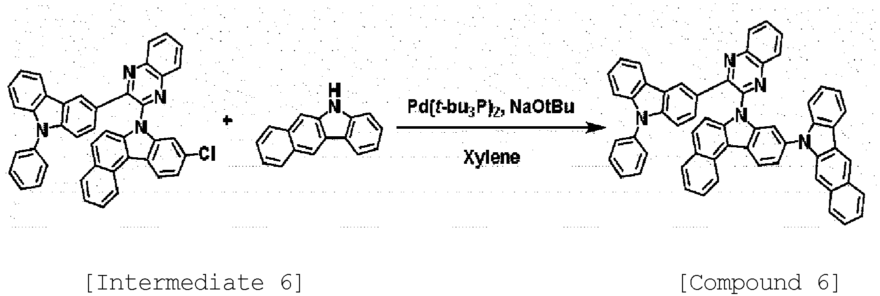

Intermediate 6 (10 g, 16.1 mmol), 5H-benzo[b]carbazole (3.7 g, 16.9 mmol) and sodium tert-butoxide (3.1 g, 32.2 mmol) were introduced to xylene (200 ml) under the nitrogen atmosphere, and the result was stirred and refluxed. After that, bis(tri-tert-butylphosphine)palladium(0) (0.2 g, 0.3 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 6 (8.1 g). (Yield 63%, MS: [M+H]⁺=802)

### Synthesis Example 7

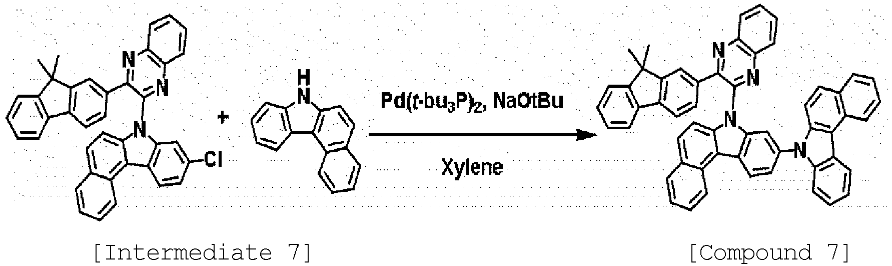

Intermediate 7 (10 g, 17.5 mmol), 7H-benzo[c]carbazole (4 g, 18.4 mmol) and sodium tert-butoxide (3.4 g, 35 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 2 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 7 (7.6 g). (Yield 58%, MS: [M+H]⁺=753)

### Synthesis Example 8

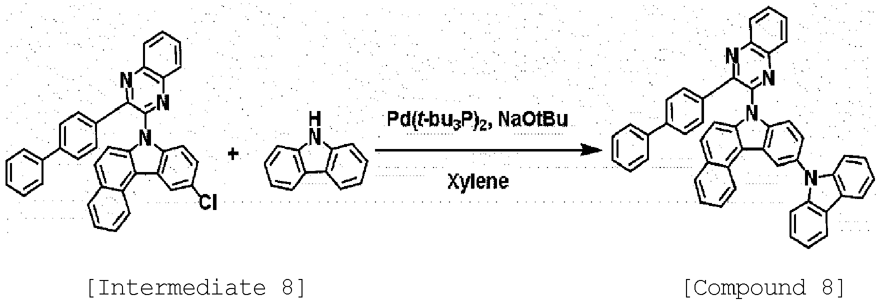

Intermediate 8 (10 g, 18.8 mmol), 9H-carbazole (3.3 g, 19.8 mmol) and sodium tert-butoxide (3.6 g, 37.7 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 8 (6.5 g). (Yield 52%, MS: [M+H]⁺=663)

### Synthesis Example 9

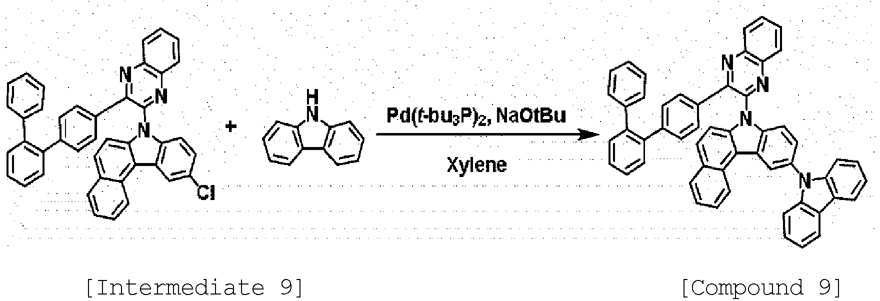

Intermediate 9 (10 g, 16.5 mmol), 9H-carbazole (2.9 g, 17.3 mmol) and sodium tert-butoxide (3.2 g, 32.9 mmol) were introduced to xylene (200 ml) under the nitrogen atmosphere, and the result was stirred and refluxed. After that, bis(tri-tert-butylphosphine)palladium(0) (0.2 g, 0.3 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 9 (6.9 g). (Yield 57%, MS: [M+H]⁺=739)

### Synthesis Example 10

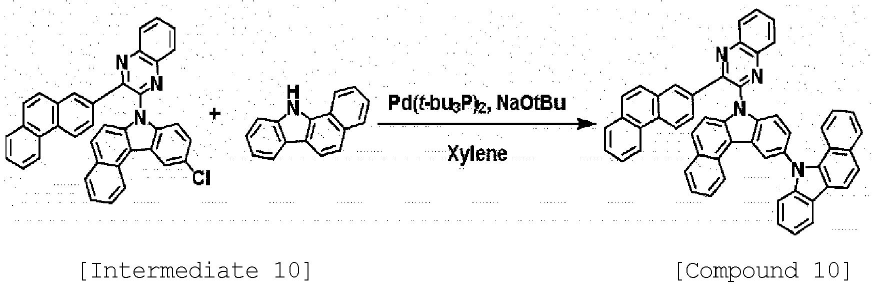

Intermediate 10 (10 g, 18 mmol), 11H-benzo[a]carbazole (4.1 g, 18.9 mmol) and sodium tert-butoxide (3.5 g, 36 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 10 (8.2 g). (Yield 62%, MS: [M+H]⁺=737)

### Synthesis Example 11

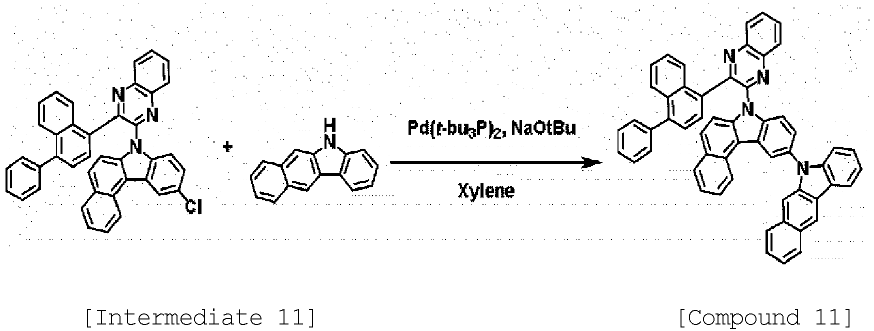

Intermediate 11 (10 g, 17.2 mmol), 5H-benzo[b]carbazole (3.9 g, 18.1 mmol) and sodium tert-butoxide (3.3 g, 34.4 mmol) were introduced to xylene (200 ml) under the nitrogen atmosphere, and the result was stirred and refluxed. After that, bis(tri-tert-butylphosphine)palladium(0) (0.2 g, 0.3 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 11 (8.3 g). (Yield 63%, MS: [M+H]⁺=763)

### Synthesis Example 12

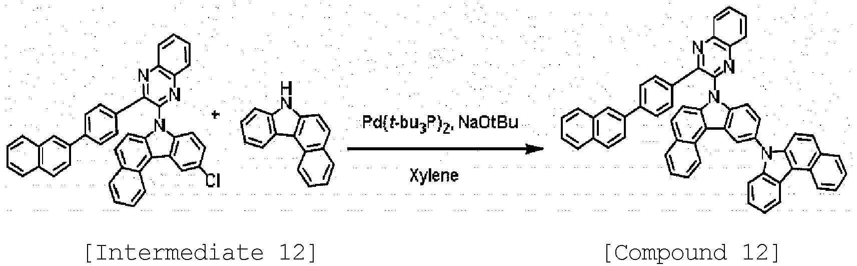

Intermediate 12 (10 g, 17.2 mmol), 7H-benzo[c]carbazole (3.9 g, 18.1 mmol) and sodium tert-butoxide (3.3 g, 34.4 mmol) were introduced to xylene (200 ml) under the nitrogen atmosphere, and the result was stirred and refluxed. After that, bis(tri-tert-butylphosphine)palladium(0) (0.2 g, 0.3 mmol) was introduced thereto. The reaction was terminated after 2 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 12 (8 g). (Yield 610, MS: [M+H]⁺=763)

### Synthesis Example 13

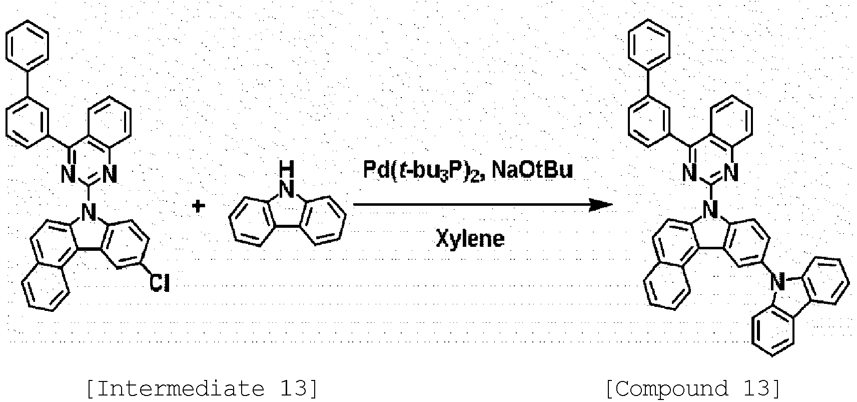

Intermediate 13 (10 g, 18.8 mmol), 9H-carbazole (3.3 g, 19.8 mmol) and sodium tert-butoxide (3.6 g, 37.7 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 13 (7.9 g). (Yield 63%, MS: [M+H]⁺=663)

### Synthesis Example 14

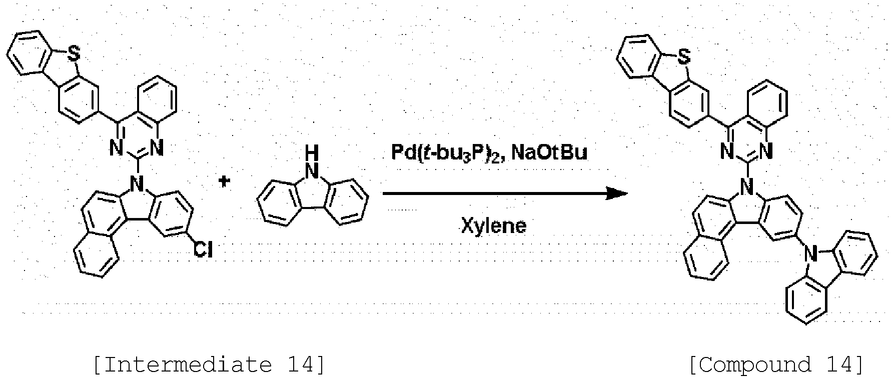

Intermediate 14 (10 g, 17.8 mmol), 9H-carbazole (3.1 g, 18.7 mmol) and sodium tert-butoxide (3.4 g, 35.6 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 14 (7.3 g). (Yield 59%, MS: [M+H]⁺=693)

### Synthesis Example 15

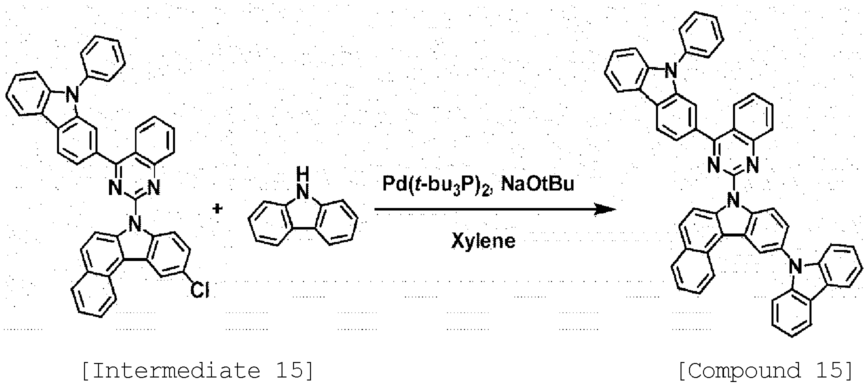

Intermediate 15 (10 g, 16.1 mmol), 9H-carbazole (2.8 g, 16.9 mmol) and sodium tert-butoxide (3.1 g, 32.2 mmol) were introduced to xylene (200 ml) under the nitrogen atmosphere, and the result was stirred and refluxed. After that, bis(tri-tert-butylphosphine)palladium(0) (0.2 g, 0.3 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 15 (7.1 g). (Yield 59%, MS: [M+H]⁺=752)

### Synthesis Example 16

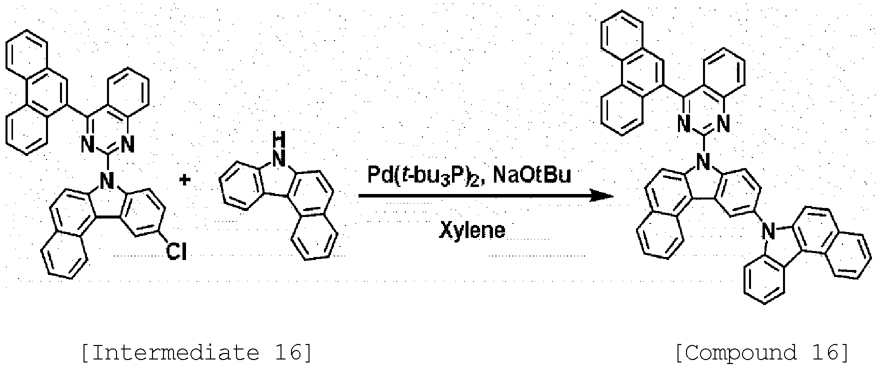

Intermediate 16 (10 g, 18 mmol), 7H-benzo[c]carbazole (4.1 g, 18.9 mmol) and sodium tert-butoxide (3.5 g, 36 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 2 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 16 (8.9 g). (Yield 67%, MS: [M+H]⁺=737)

### Synthesis Example 17

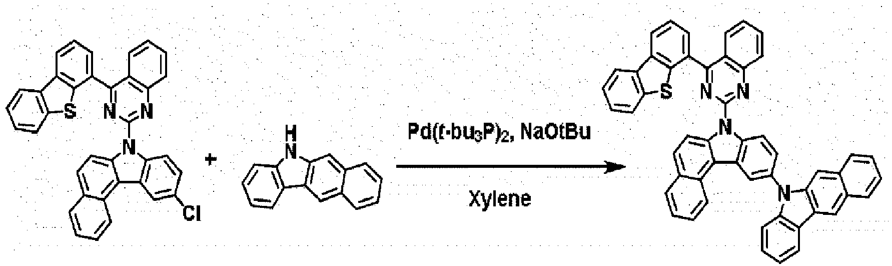

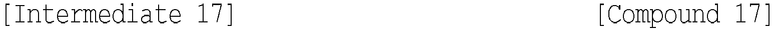

Intermediate 17 (10 g, 17.8 mmol), 5H-benzo[b]carbazole (4.1 g, 18.7 mmol) and sodium tert-butoxide (3.4 g, 35.6 mmol) were introduced to xylene (200 ml) under the nitrogen atmosphere, and the result was stirred and refluxed. After that, bis(tri-tert-butylphosphine)palladium(0) (0.2 g, 0.4 mmol) was introduced thereto. The reaction was terminated after 3 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 17 (7 g). (Yield 53%, MS: [M+H]⁺=743)

### Synthesis Example 18

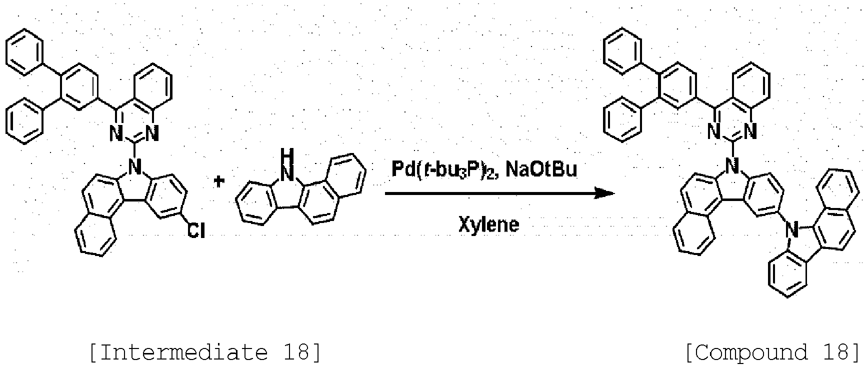

Intermediate 18 (10 g, 16.5 mmol), 11H-benzo[a]carbazole (3.8 g, 17.3 mmol) and sodium tert-butoxide (3.2 g, 32.9 mmol) were introduced to xylene (200 ml) under the nitrogen atmosphere, and the result was stirred and refluxed. After that, bis(tri-tert-butylphosphine)palladium(0) (0.2 g, 0.3 mmol) was introduced thereto. The reaction was terminated after 2 hours, and the result was cooled to room temperature and vacuumed to remove the solvent. After that, the compound was completely dissolved in chloroform again, and washed twice with water. Then, the organic layer was separated, treated with anhydrous magnesium sulfate, then filtered, and the filtrate was vacuum distilled. The concentrated compound was purified using silica gel column chromatography to obtain Compound 18 (7.5 g). (Yield 58%, MS: [M+H]⁺=789)

### Comparative Example 1

A glass substrate on which indium tin oxide (ITO) was coated as a thin film to a thickness of 1,000 Å was placed in detergent-dissolved distilled water and ultrasonic cleaned. Herein, a product of Fischer Co. was used as the detergent, and as the distilled water, distilled water filtered twice with a filter manufactured by Millipore Co. was used. After the ITO was cleaned for 30 minutes, ultrasonic cleaning was repeated twice using distilled water for 10 minutes. After the cleaning with distilled water was finished, the substrate was ultrasonic cleaned with solvents of isopropyl alcohol, acetone and methanol, then dried, and then transferred to a plasma cleaner. In addition, the substrate was cleaned for 5 minutes using oxygen plasma, and then transferred to a vacuum depositor.

On the transparent ITO electrode prepared as above, the following HI-1 compound was formed to a thickness of 1150 Å as a hole injection layer while p-doping the following A-1 compound thereto in a concentration of 1.5%. On the hole injection layer, the following HT-1 compound was vacuum deposited to form a hole transfer layer having a film thickness of 1000 Å. Subsequently, the HT-2 Compound was vacuum deposited to a film thickness of 500 Å on the hole transfer layer to form a hole transfer auxiliary layer. On the hole transfer auxiliary layer, the following EB-1 compound was vacuum deposited to a film thickness of 150 Å to form an electron blocking layer. Then, on the EB-1 deposited film, the following RH-1 compound and the following Dp-8 compound were vacuum deposited in a weight ratio of 98:2 to form a red light emitting layer having a thickness of 400 Å. On the light emitting layer, a hole blocking layer was formed by vacuum depositing the following HB-1 compound to a film thickness of 30 Å. Subsequently, an electron injection and transfer layer was formed on the hole blocking layer to a thickness of 300 Å by vacuum depositing the following ET-1 Compound and the following LiQ compound in a weight ratio of 2:1. On the electron injection and transfer layer, a cathode was formed by consecutively depositing lithium fluoride (LiF) and aluminum to a thickness of 12 Å and a thickness of 1,000 Å, respectively.

In the above-mentioned process, the deposition rates of the organic materials were maintained at 0.4 Å/sec to 0.7 Å/sec, the deposition rates of the lithium fluoride and the aluminum of the cathode were maintained at 0.3 Å/sec and 2 Å/sec, respectively, and the degree of vacuum during the deposition was maintained at 2×10⁻⁷ torr to 5×10⁻⁶ torr, and as a result, an organic light emitting device was manufactured.

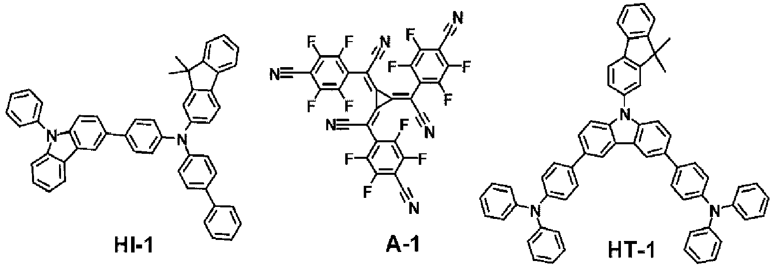

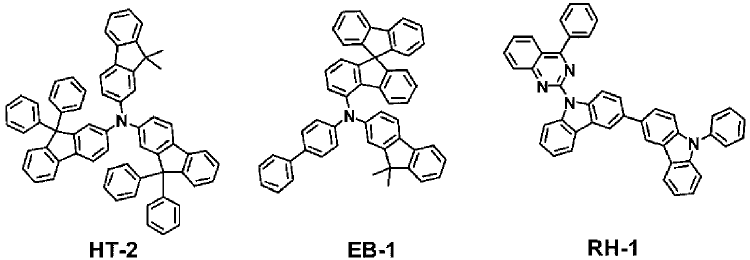

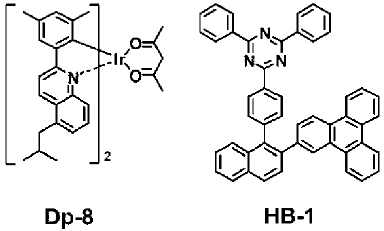

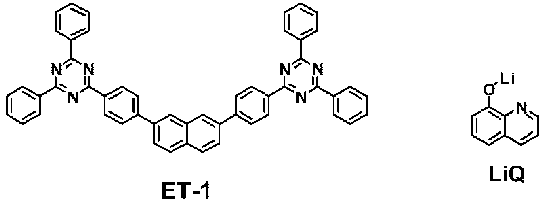

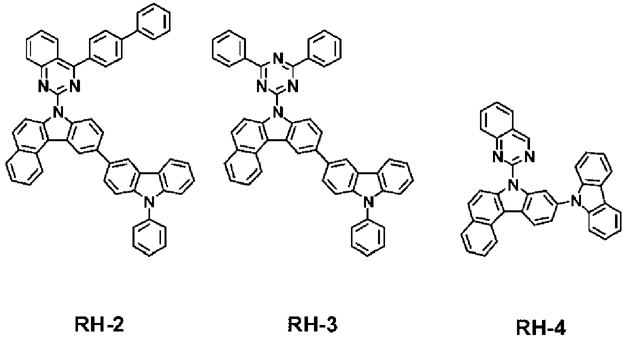

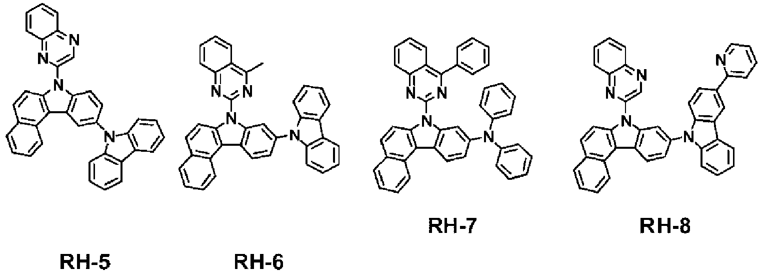

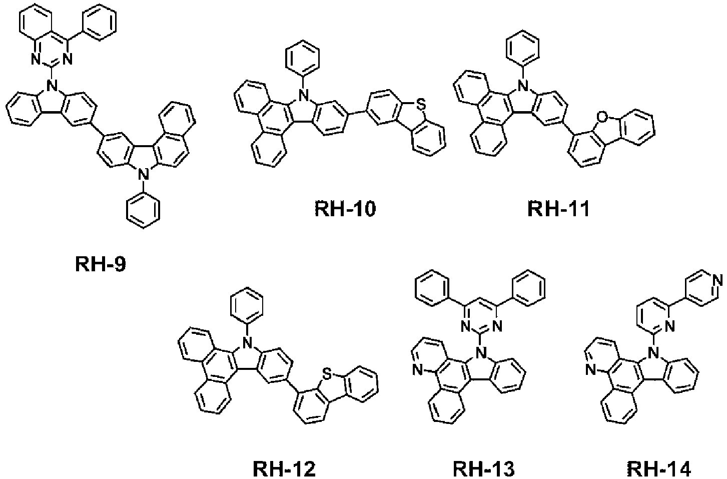

### Example 1 to Example 18

Organic light emitting devices were manufactured in the same manner as the organic light emitting device of Comparative Example 1 except that compounds described in the following Table 1 were used instead of RH-1.

### Comparative Example 2 to Comparative Example 14

Organic light emitting devices were manufactured in the same manner as the organic light emitting device of Comparative Example 1 except that compounds described in the following Table 1 were used instead of RH-1.

When applying a current of 10 mA/cm² to each of the organic light emitting devices manufactured in Example 1 to Example 18 and Comparative Example 1 to Comparative Example 14, voltage, efficiency and lifetime were measured, and the results are shown in the following Table 1. T95 means time taken for luminance decreasing to 95% from initial luminance (5000 nit).

**[Table 1]**

| | Light Emitting Layer | Driving Voltage (V) | Efficiency (cd/A) | Lifetime (T95) |
|---|---|---|---|---|
| Comparative Example1 | RH-1 | 4.8 | 33 | 221 |
| Example 1 | Compound 1 | 4.1 | 43 | 292 |
| Example 2 | Compound 2 | 4.4 | 40 | 347 |
| Example 3 | Compound 3 | 4.0 | 44 | 271 |
| Example 4 | Compound 4 | 4.1 | 42 | 283 |
| Example 5 | Compound 5 | 4.3 | 39 | 301 |
| Example 6 | Compound 6 | 4.1 | 43 | 284 |
| Example 7 | Compound 7 | 4.1 | 44 | 257 |
| Example 8 | Compound 8 | 4.2 | 41 | 291 |
| Example 9 | Compound 9 | 4.1 | 45 | 278 |
| Example 10 | Compound 10 | 4.0 | 40 | 283 |
| Example 11 | Compound 11 | 4.2 | 44 | 261 |
| Example 12 | Compound 12 | 4.1 | 42 | 252 |
| Example 13 | Compound 13 | 4.3 | 38 | 310 |
| Example 14 | Compound 14 | 4.4 | 36 | 297 |
| Example 15 | Compound 15 | 4.2 | 39 | 307 |
| Example 16 | Compound 16 | 4.3 | 36 | 311 |
| Example 17 | Compound 17 | 4.2 | 37 | 302 |
| Example 18 | Compound 18 | 4.4 | 38 | 291 |
| Comparative Example 2 | RH-2 | 4.7 | 34 | 231 |
| Comparative Example 3 | RH-3 | 4.4 | 37 | 173 |
| Comparative Example 4 | RH-4 | 4.9 | 28 | 78 |
| Comparative Example 5 | RH-5 | 4.9 | 30 | 31 |
| Comparative Example 6 | RH-6 | 5.0 | 24 | 57 |
| Comparative Example 7 | RH-7 | 5.4 | 31 | 56 |
| Comparative Example 8 | RH-8 | 4.9 | 29 | 34 |
| Comparative Example 9 | RH-9 | 4.8 | 33 | 208 |
| Comparative Example 10 | RH-10 | 5.2 | 14 | 31 |
| Comparative Example 11 | RH-11 | 5.1 | 17 | 38 |
| Comparative Example 12 | RH-12 | 5.1 | 19 | 34 |
| Comparative Example 13 | RH-13 | 4.9 | 28 | 135 |
| Comparative Example 14 | RH-14 | 5.2 | 13 | 11 |

When examining Table 1 presenting the results of the experiments, it was identified that the compound of the present disclosure was capable of improving light emission efficiency, driving voltage and lifetime properties in driving a red light emitting device.

Specifically, in Comparative Examples 2, 3 and 9, the substituent that is not hydrogen among the substituents corresponding to substituents R7 to R10 of the present disclosure is carbazole, however, bonding types of the carbazole are different. While Chemical Formula A of the present disclosure has N of the carbazole directly bonding to the core, Compounds RH-2, RH-3 and RH-9 have benzene of the carbazole bonding to the core structure. In Comparative Example 7, the substituent that is not hydrogen among the substituents corresponding to substituents R7 to R10 is an amine group.

It was seen that Examples 1 to 18 had properties of low voltage, high efficiency and long lifetime compared to Comparative Examples 2, 3, 7 and 9.

In RH-4, RH-5, RH-6 and RH-8 used in Comparative Examples 4, 5, 6 and 8, respectively, the substituent corresponding to R of Chemical Formula 1 of the present disclosure is hydrogen or an alkyl group. Meanwhile, in the present disclosure, R is an aryl group or a heteroaryl group. It was seen that Examples 1 to 18 had properties of low voltage, high efficiency and long lifetime compared to Comparative Examples 4, 5, 6 and 8.

Comparative Examples 10 to 14 used Compounds RH-10 to RH-14 in which R1 to R6 form an additional aromatic or heteroring. In the present disclosure, R1 to R6 did not form an additional ring. It was seen that Examples 1 to 18 had properties of low voltage, high efficiency and long lifetime compared to Comparative Examples 10 to 14.

When comparing the experimental results between the compounds of the present disclosure and the compounds of the comparative examples, it was seen that, in the compounds of the present disclosure, electrons and holes were well-balanced in the red device structure, which was advantageous for exciton formation and allowed favorable energy transfer to the dopant, and as a result, driving voltage, efficiency and lifetime properties were all improved.

## Claims

1. A compound represented by the following Chemical Formula 1: 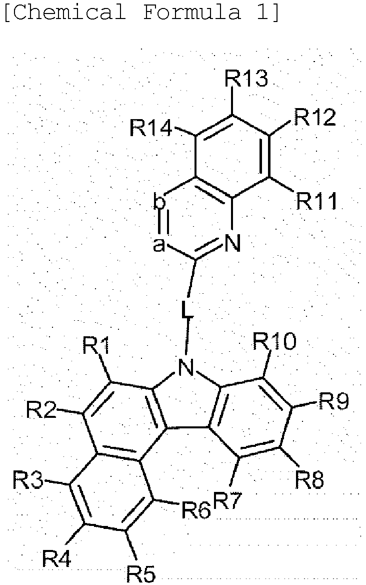
wherein, in Chemical Formula 1,
L is a direct bond, a substituted or unsubstituted arylene group, or a substituted or unsubstituted heteroarylene group;
a is CR;
b is N;;
R is a substituted or unsubstituted aryl group, or a substituted or unsubstituted heteroaryl group;
R1 to R6 and R11 to R14 are the same as or different from each other, and each independently hydrogen, a nitrile group, a halogen group, a substituted or unsubstituted alkyl group, a substituted or unsubstituted silyl group, a substituted or unsubstituted aryl group, or a substituted or unsubstituted heteroaryl group; and
at least one of R7 to R10 is the following Chemical Formula A, and the rest are hydrogen, 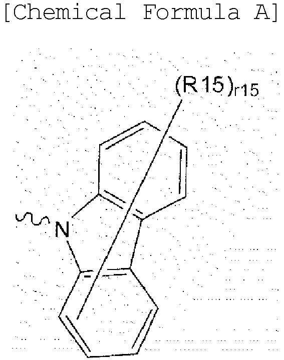
in Chemical Formula A,
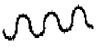 means a bond with R7 to R10;
R15 is a nitrile group, a halogen group, a substituted or unsubstituted alkyl group, a substituted or unsubstituted silyl group, a substituted or unsubstituted aryl group, or a substituted or unsubstituted heteroaryl group, or adjacent groups bond to each other to form a substituted or unsubstituted ring; and
r15 is an integer of 0 to 8, and when r15 is 2 or greater, R15s are the same as or different from each other.

2. The compound of Claim 1, wherein R is an unsubstituted aryl group having 6 to 30 carbon atoms; an aryl group having 6 to 30 carbon atoms substituted with an aryl group; an aryl group having 6 to 30 carbon atoms substituted with an alkyl group; or a heteroaryl group having 3 to 30 carbon atoms that is unsubstituted or is substituted with an aryl group.

3. The compound of Claim 1, wherein R1 to R6 and R11 to R14 are hydrogen.

4. The compound of Claim 1, wherein L is a direct bond.

5. The compound of Claim 1, wherein Chemical Formula 1 is any one selected from among the following compounds:

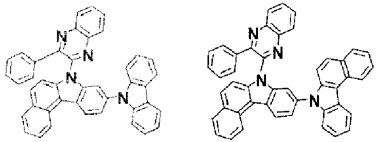

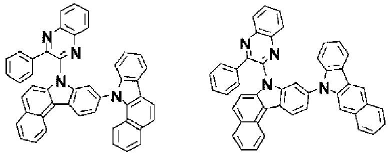

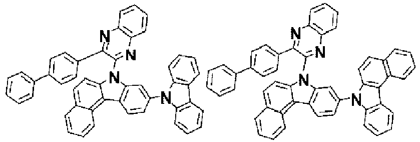

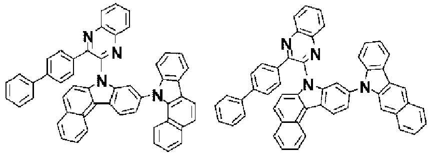

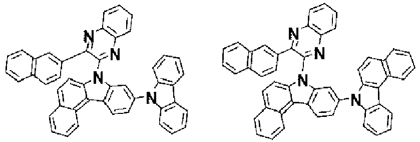

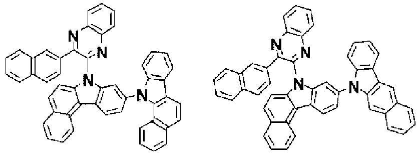

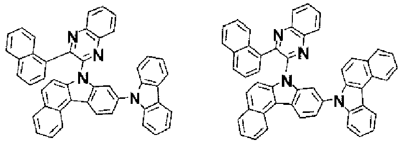

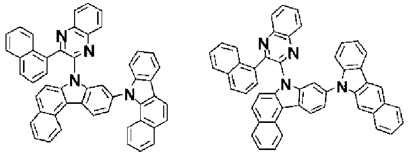

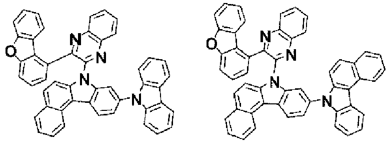

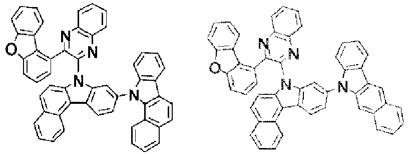

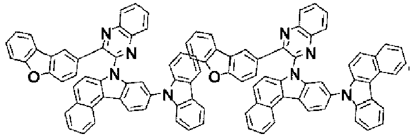

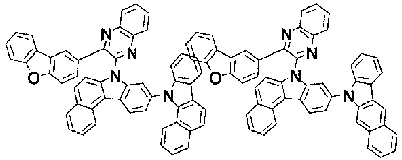

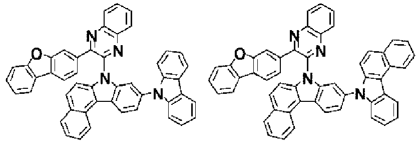

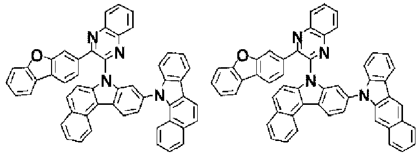

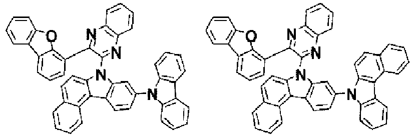

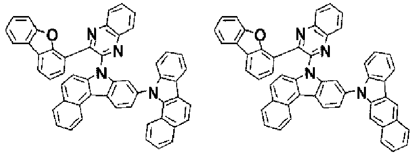

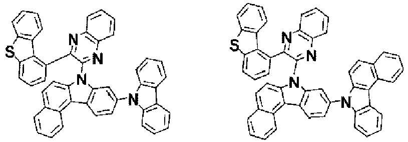

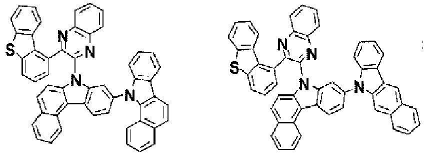

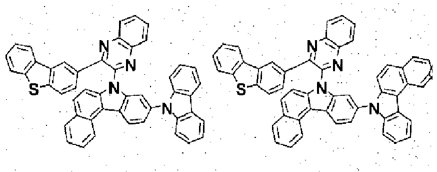

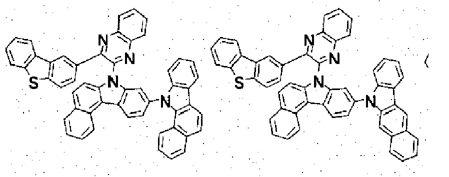

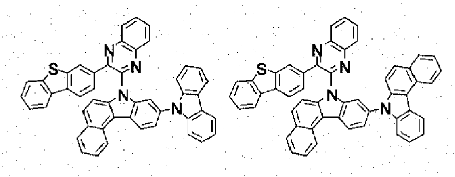

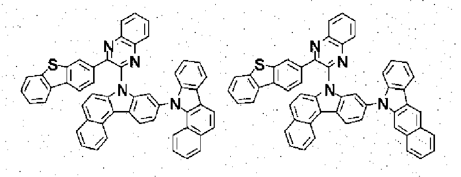

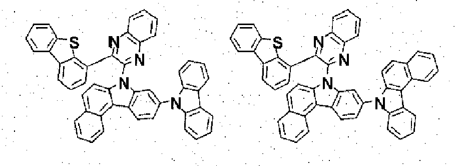

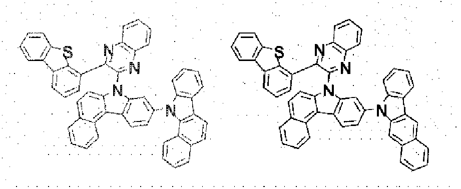

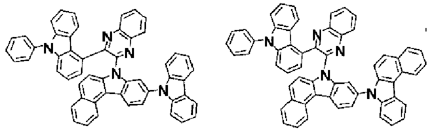

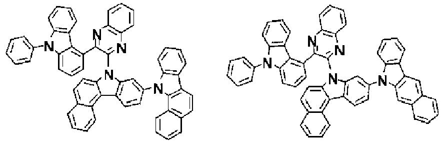

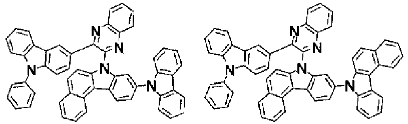

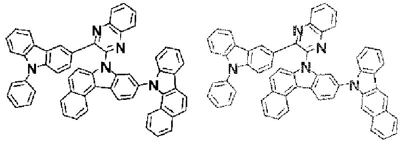

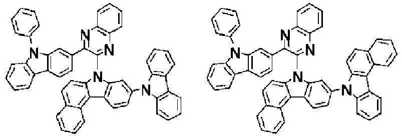

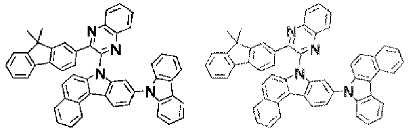

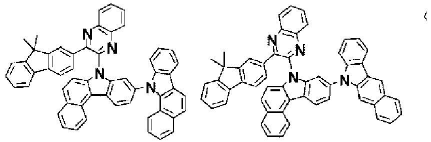

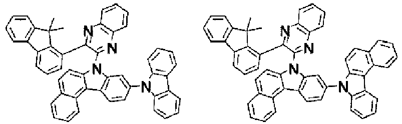

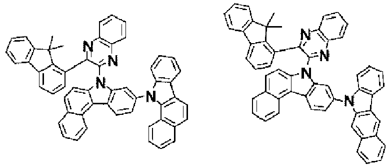

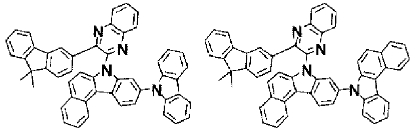

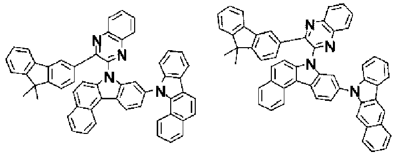

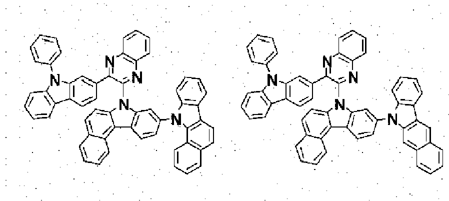

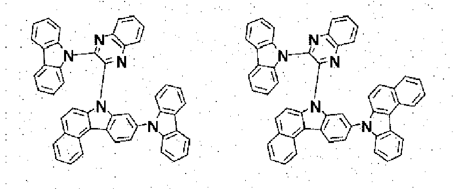

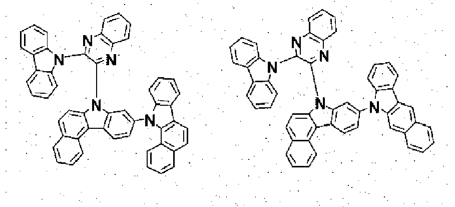

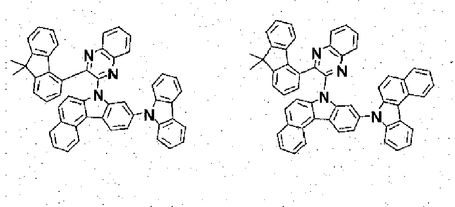

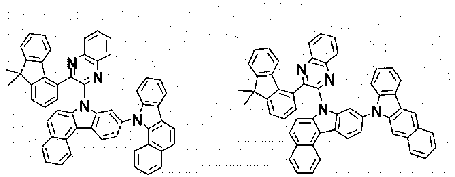

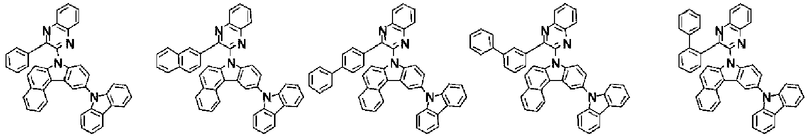

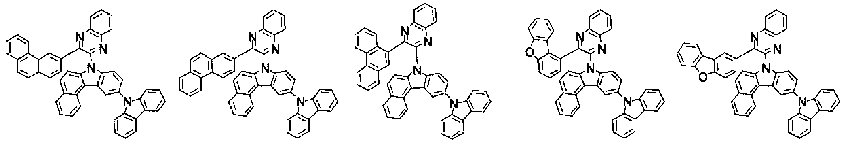

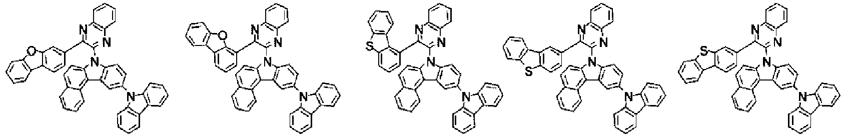

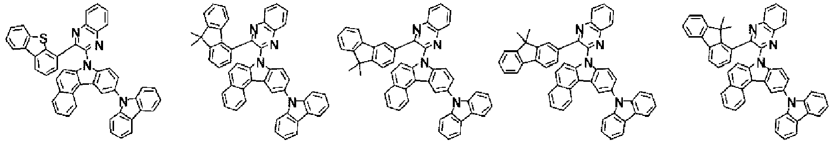

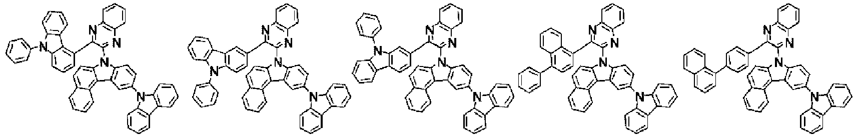

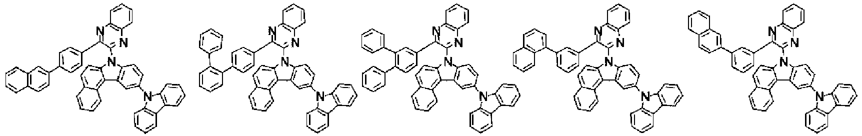

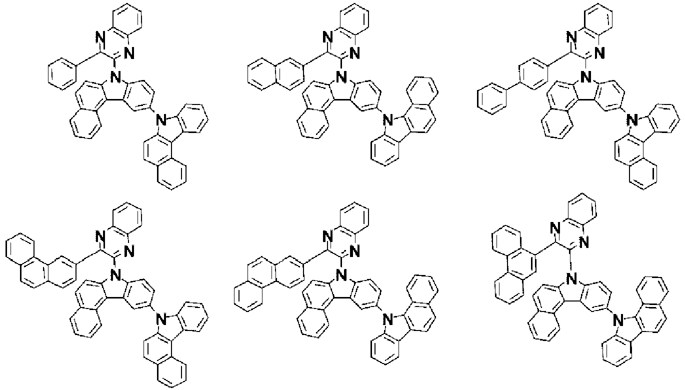

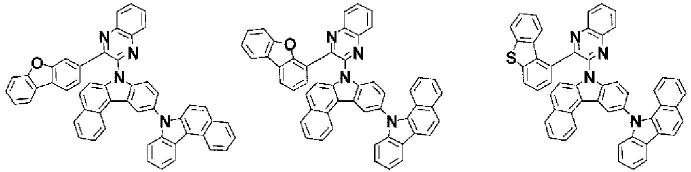

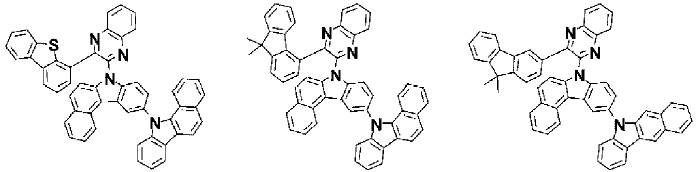

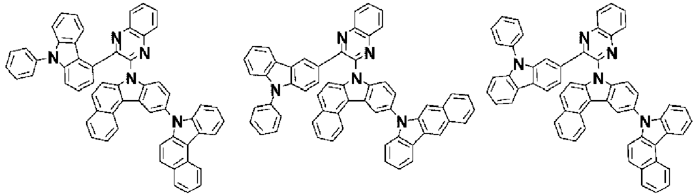

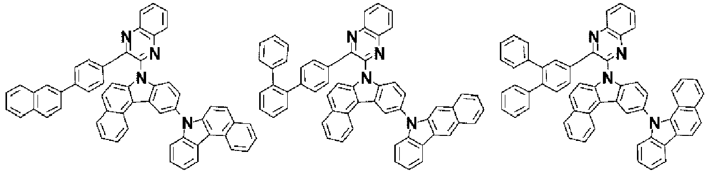

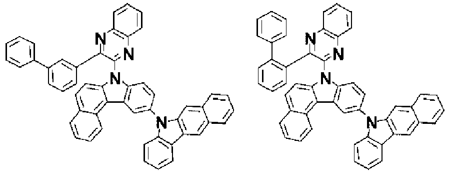

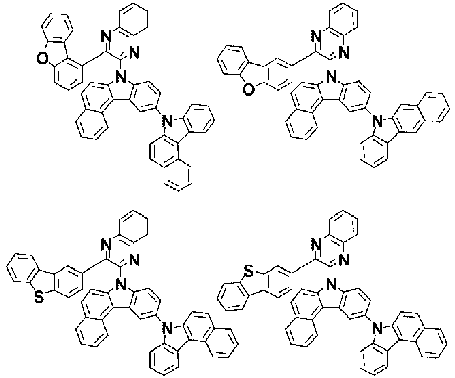

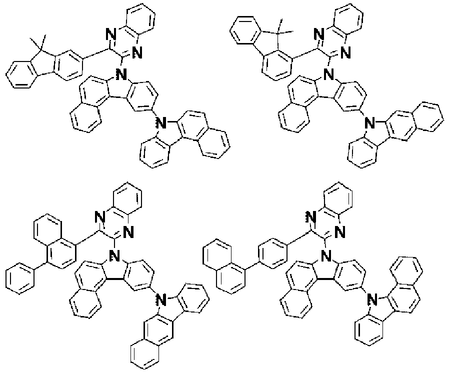

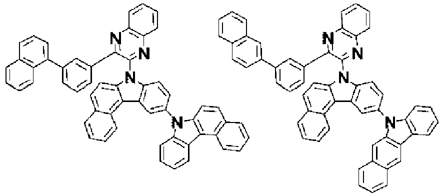

6. An organic light emitting device comprising:
a first electrode;
a second electrode provided opposite to the first electrode; and
one, two or more organic material layers provided between the first electrode and the second electrode,
wherein one or more layers of the organic material layers include the compound of any one of Claims 1 to 5.

7. The organic light emitting device of Claim 6, wherein the organic material layer includes a light emitting layer, and the light emitting layer includes the compound.

## Patentansprüche

1. Verbindung, dargestellt durch die folgende chemische Formel 1: 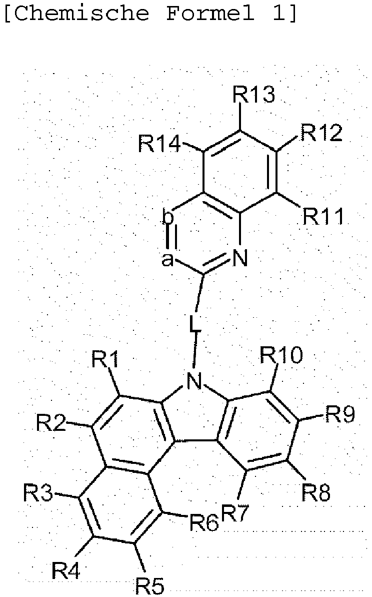
worin in der chemischen Formel 1
L eine direkte Bindung, eine substituierte oder unsubstituierte Arylengruppe oder eine substituierte oder unsubstituierte Heteroarylengruppe ist;
a CR ist;
b N ist;
R eine substituierte oder unsubstituierte Arylgruppe oder eine substituierte oder unsubstituierte Heteroarylgruppe ist;
R1 bis R6 und R11 und R14 gleich oder voneinander verschieden sind und jeweils unabhängig Wasserstoff, eine Nitrilgruppe, eine Halogengruppe, eine substituierte oder unsubstituierte Alkylgruppe, eine substituierte oder unsubstituierte Silylgruppe, eine substituierte oder unsubstituierte Arylgruppe oder eine substituierte oder unsubstituierte Heteroarylgruppe darstellen; und
zumindest eines von R7 bis R10 die folgende chemische Formel A ist und die verbleibenden Wasserstoff sind, 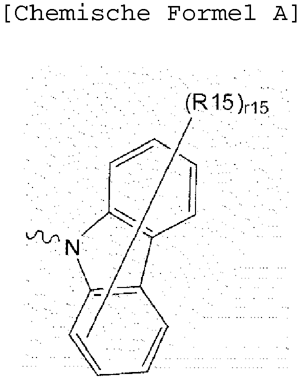
worin in der chemischen Formel A
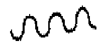 eine Bindung mit R7 bis R10 bezeichnet;
R15 eine Nitrilgruppe, eine Halogengruppe, eine substituierte oder unsubstituierte Alkylgruppe, eine substituierte oder unsubstituierte Silylgruppe, eine substituierte oder unsubstituierte Arylgruppe oder eine substituierte oder unsubstituierte Heteroarylgruppe ist, oder benachbarte Gruppen aneinander binden, um einen substituierten oder unsubstituierten Ring zu bilden; und
r15 eine ganze Zahl von 0 bis 8 ist, und wenn r15 2 oder größer ist, die R15 gleich oder verschieden sind.

2. Verbindung gemäß Anspruch 1, worin R eine unsubstituierte Arylgruppe mit 6 bis 30 Kohlenstoffatomen; eine Arylgruppe mit 6 bis 30 Kohlenstoffatomen, die mit einer Arylgruppe substituiert ist; eine Arylgruppe mit 6 bis 30 Kohlenstoffatomen, die mit einer Alkylgruppe substituiert ist; oder eine Heteroarylgruppe mit 3 bis 30 Kohlenstoffatomen, die unsubstituiert ist oder mit einer Arylgruppe substituiert ist, ist.

3. Verbindung gemäß Anspruch 1, worin R1 bis R6 und R11 bis R14 Wasserstoff sind.

4. Verbindung gemäß Anspruch 1, worin L eine direkte Bindung ist.

5. Verbindung gemäß Anspruch 1, worin die chemische Formel 1 irgendeine ist, die unter den folgenden Verbindungen ausgewählt ist:

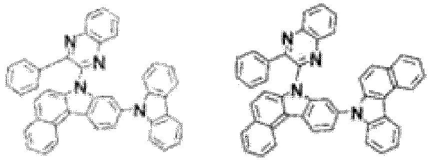

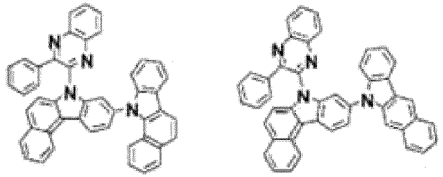

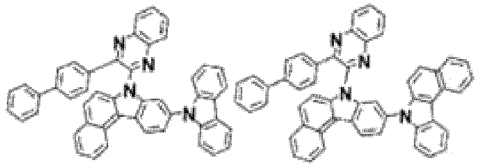

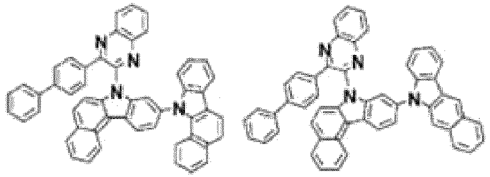

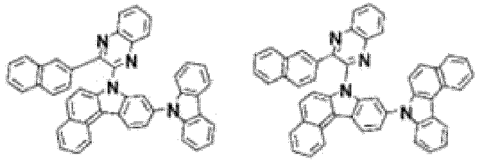

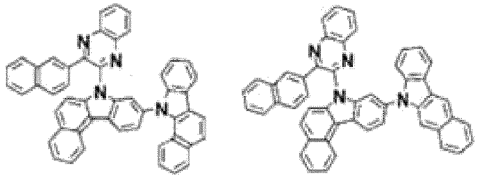

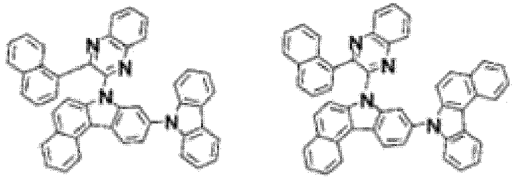

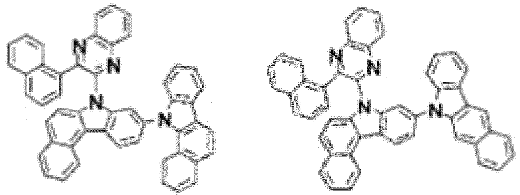

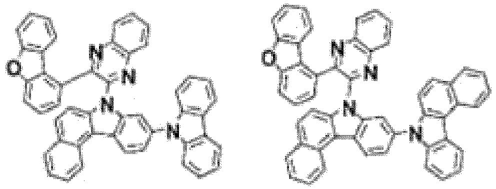

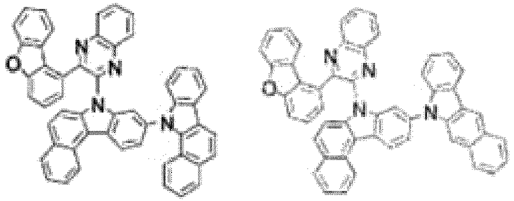

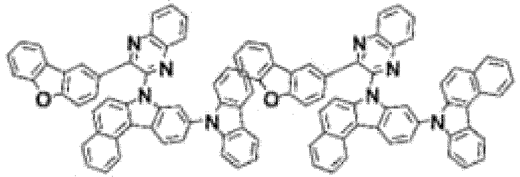

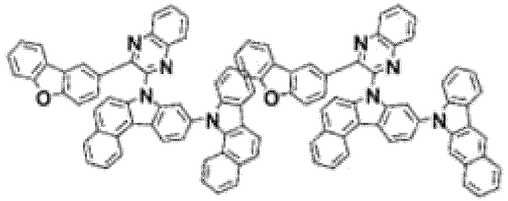

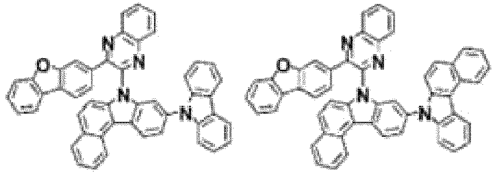

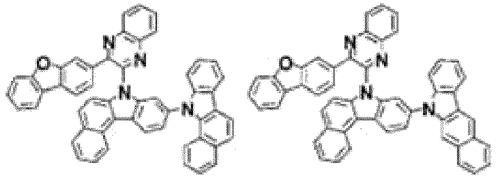

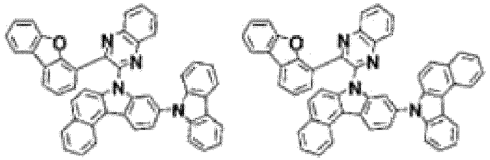

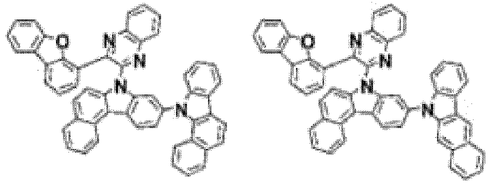

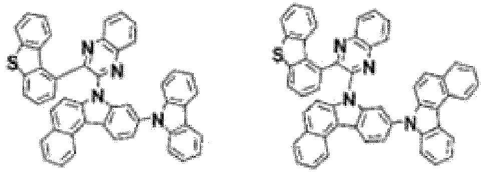

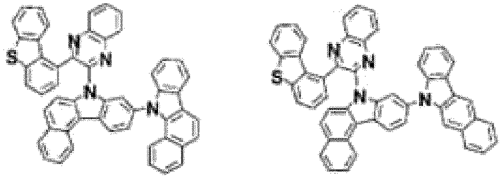

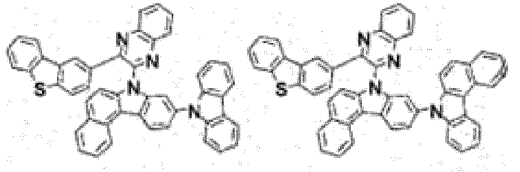

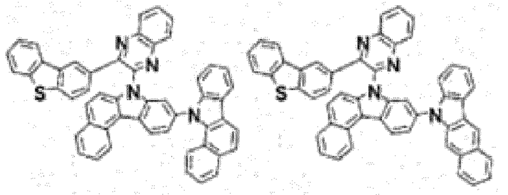

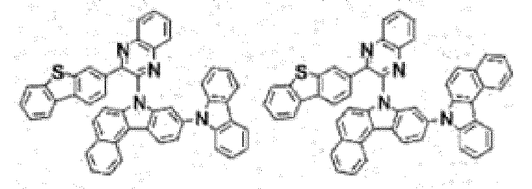

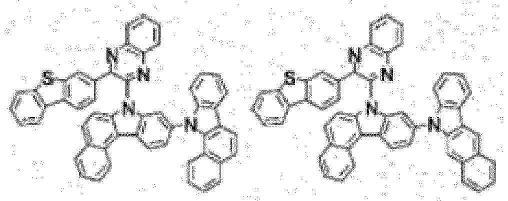

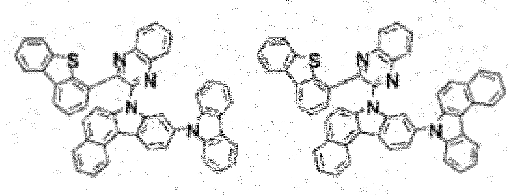

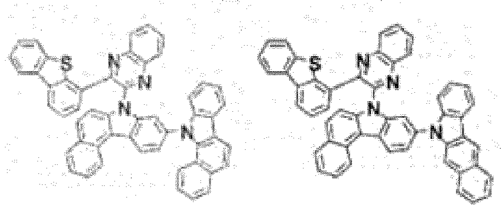

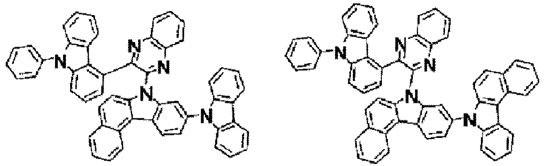

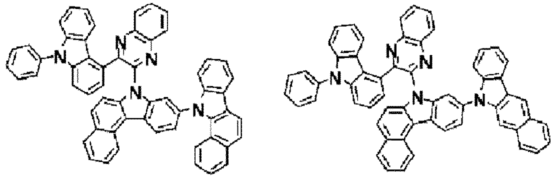

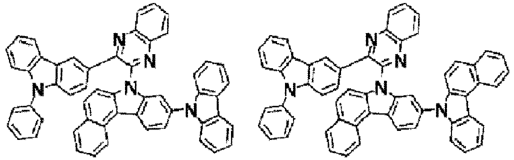

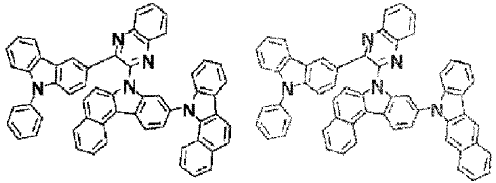

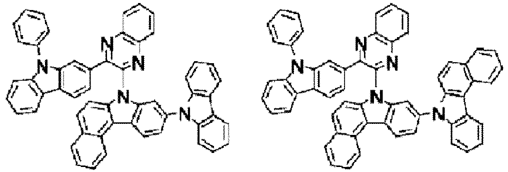

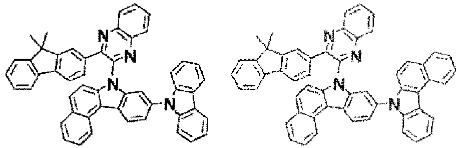

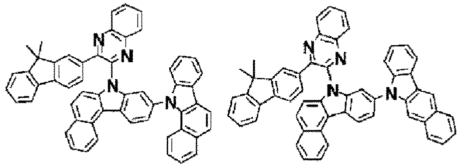

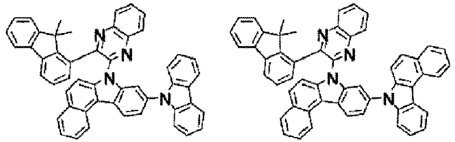

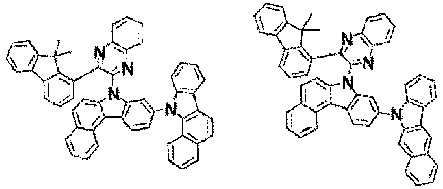

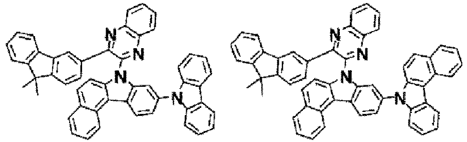

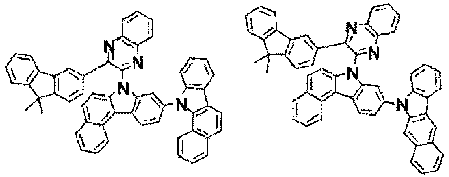

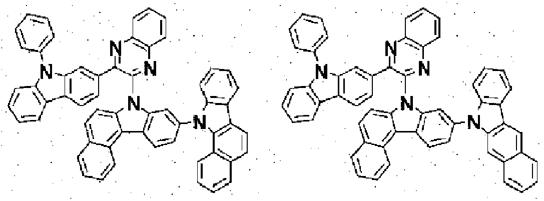

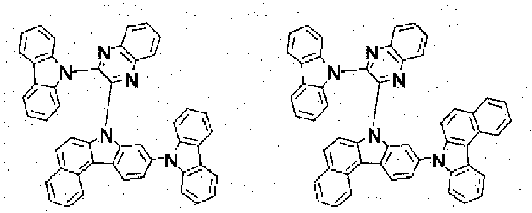

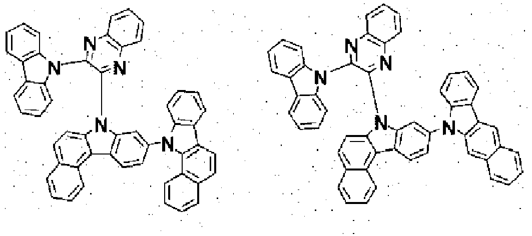

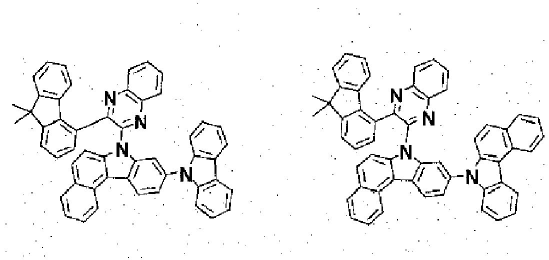

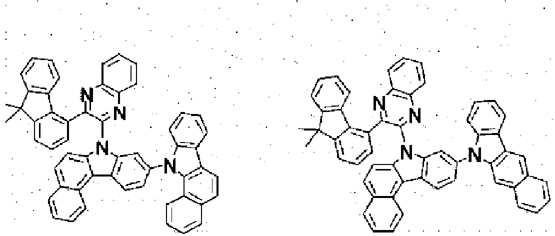

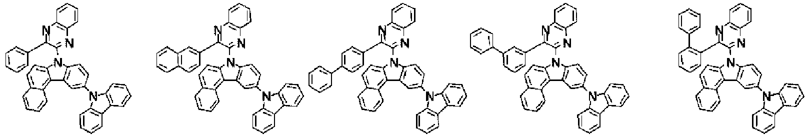

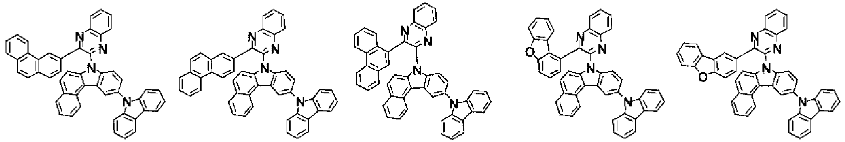

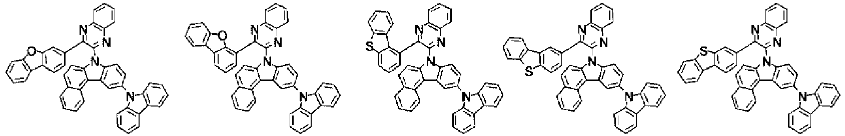

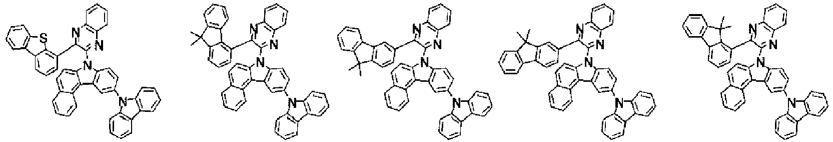

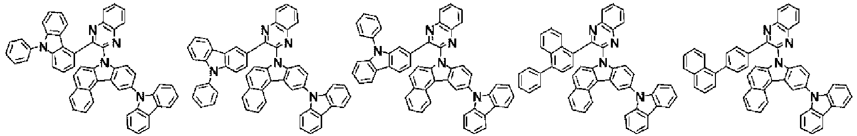

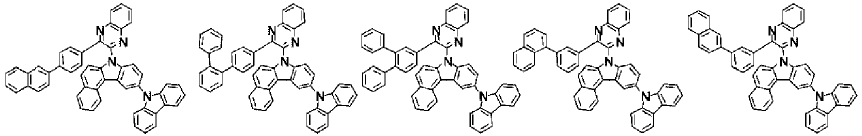

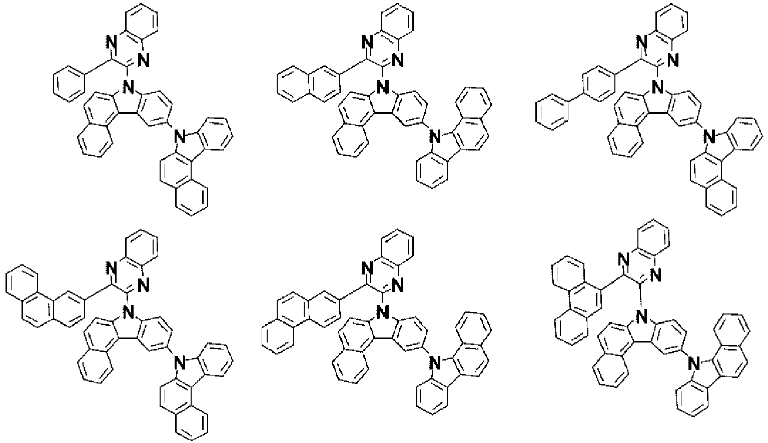

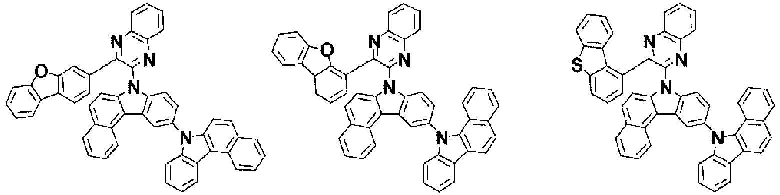

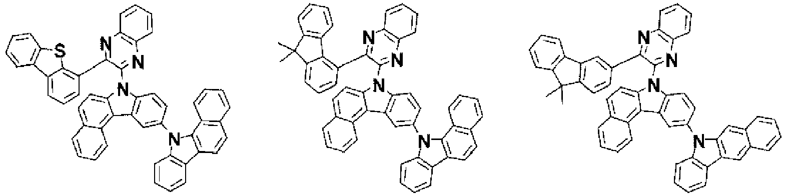

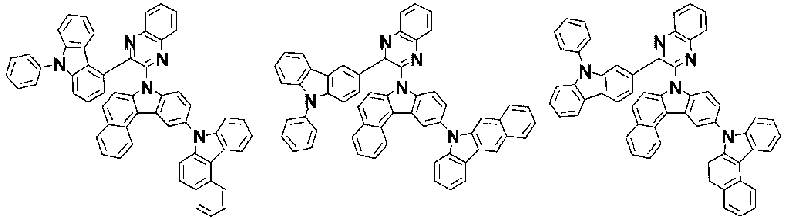

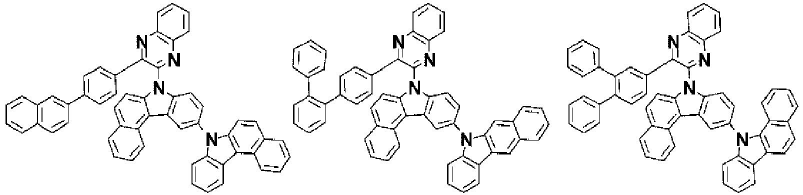

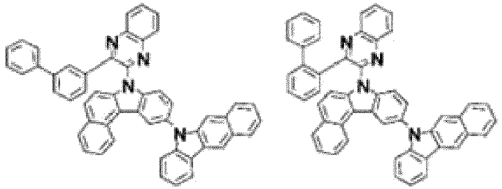

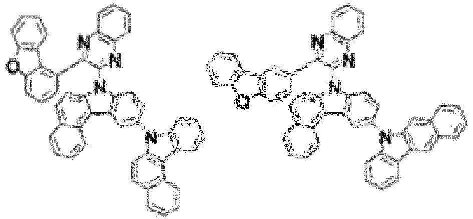

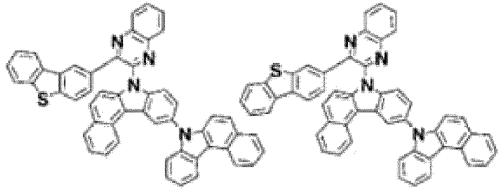

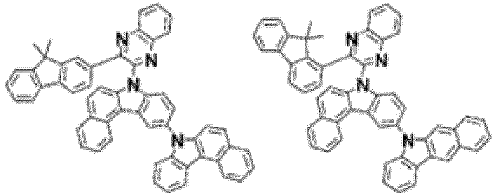

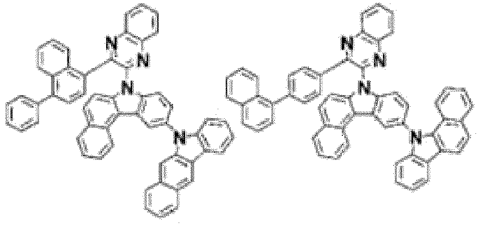

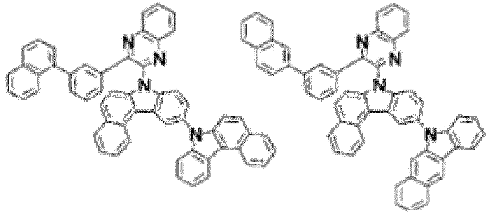

6. Organische lichtemittierende Vorrichtung, umfassend:
eine erste Elektrode;
eine gegenüberliegend zu der ersten Elektrode vorgesehene zweite Elektrode; und
eine, zwei oder mehr zwischen der ersten Elektrode und der zweiten Elektrode vorgesehene organische Materialschichten,
worin eine oder mehr Schichten der organischen Materialschichten die Verbindung gemäß irgendeinem der Ansprüche 1 bis 5 umfasst.

7. Organische lichtemittierende Vorrichtung gemäß Anspruch 6, worin die organische Materialschicht eine lichtemittierende Schicht umfasst und die lichtemittierende Schicht die Verbindung umfasst.

## Revendications

1. Composé représenté par la formule chimique 1 suivante : 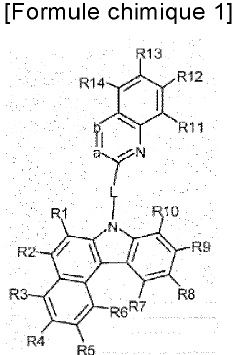
dans lequel, dans la formule chimique 1,
L est une liaison directe, un groupe arylène substitué ou non substitué ou un groupe hétéroarylène substitué ou non substitué ;
a est du CR ;
b est du N ;;
R est un groupe aryle substitué ou non substitué ou un groupe hétéroaryle substitué ou non substitué ;
R1 à R6 et R11 à R14 sont identiques ou différents les uns des autres, et chacun indépendamment de l'hydrogène, un groupe nitrile, un groupe halogène, un groupe alkyle substitué ou non substitué, un groupe silyle substitué ou non substitué, un groupe aryle substitué ou non substitué ou un groupe hétéroaryle substitué ou non substitué ; et
au moins un de R7 à R10 est la formule chimique A suivante, et le reste est de l'hydrogène, 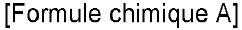 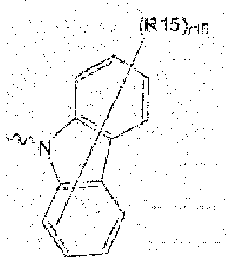
dans la formule chimique A,
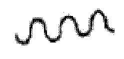 désigne une liaison avec R7 à R10 ;
R15 est un groupe nitrile, un groupe halogène, un groupe alkyle substitué ou non substitué, un groupe silyle substitué ou non substitué, un groupe aryle substitué ou non substitué ou un groupe hétéroaryle substitué ou non substitué, ou des groupes adjacents se lient entre eux pour former un cycle substitué ou non substitué ; et
r15 est un nombre entier de 0 à 8, et lorsque r15 vaut 2 ou plus, les R15 sont identiques ou différents les uns des autres.

2. Composé selon la revendication 1, dans lequel R est un groupe aryle non substitué présentant 6 à 30 atomes de carbone ; un groupe aryle présentant 6 à 30 atomes de carbone substitués par un groupe aryle ; un groupe aryle présentant 6 à 30 atomes de carbone substitués par un groupe alkyle ; ou un groupe hétéroaryle présentant 3 à 30 atomes de carbone qui est non substitué ou est substitué par un groupe aryle.

3. Composé selon la revendication 1, dans lequel R1 à R6 et R11 à R14 sont de l'hydrogène.

4. Composé selon la revendication 1, dans lequel L est une liaison directe.

5. Composé selon la revendication 1, dans lequel la formule chimique 1 est l'un quelconque sélectionné parmi les composés suivants :

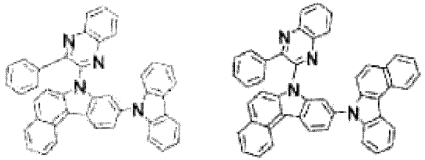

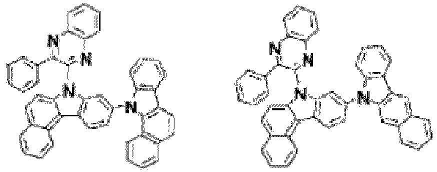

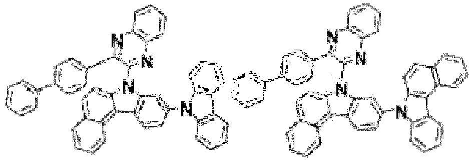

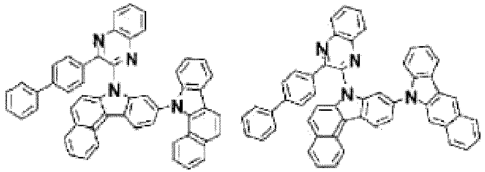

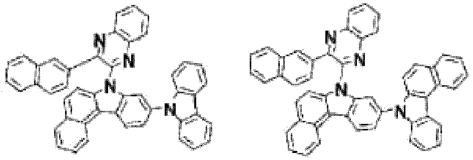

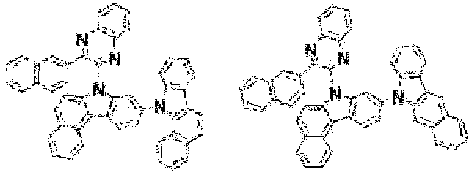

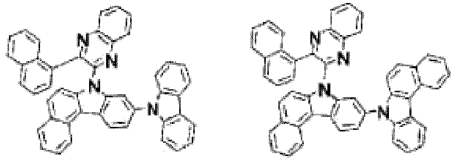

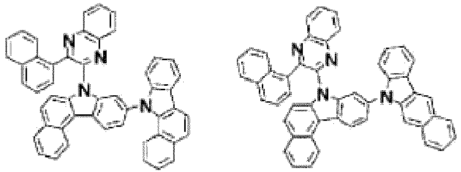

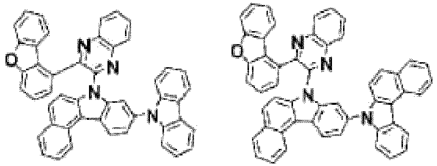

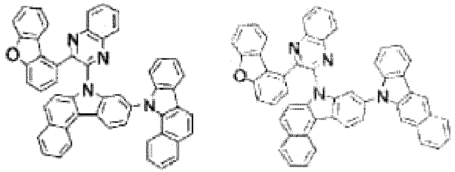

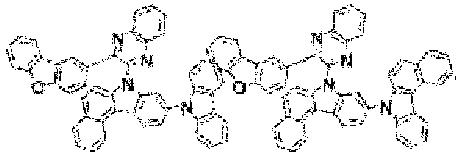

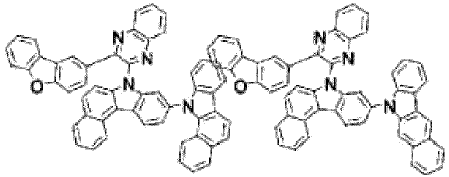

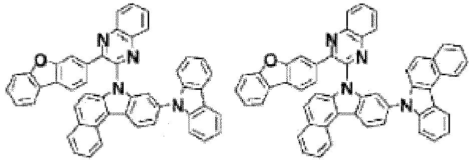

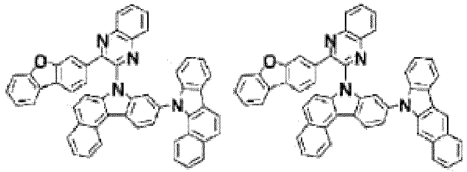

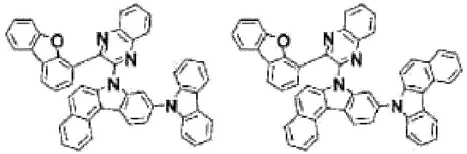

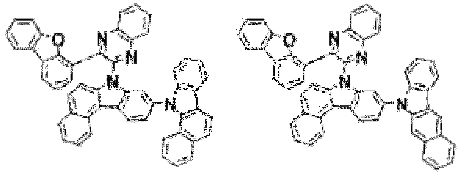

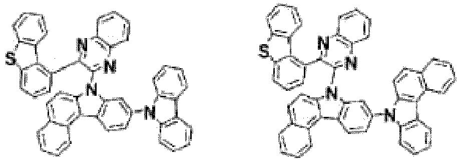

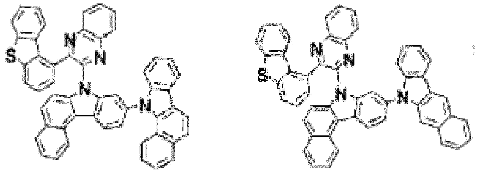

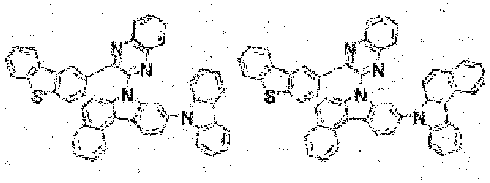

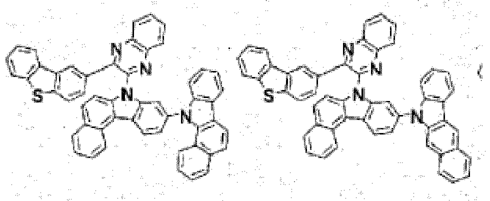

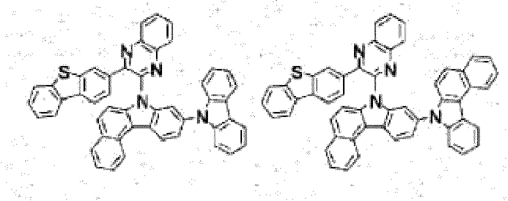

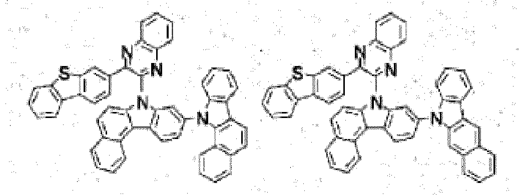

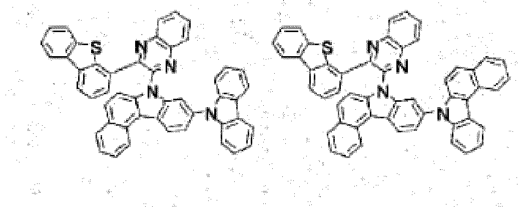

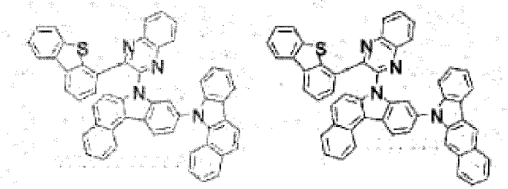

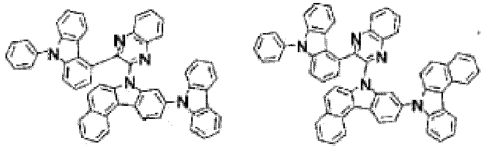

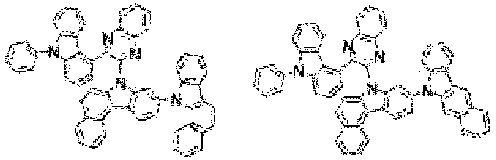

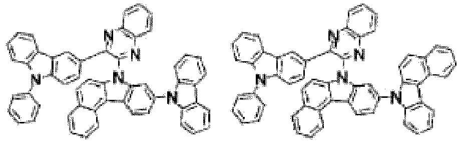

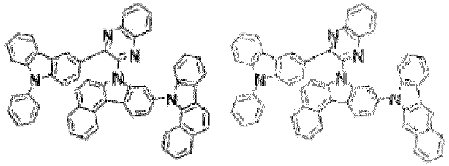

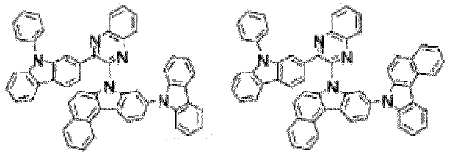

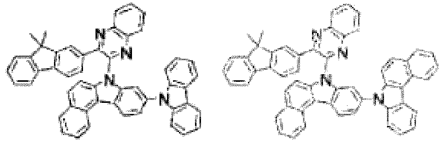

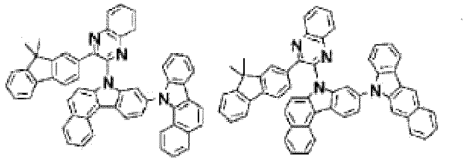

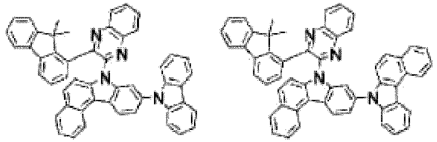

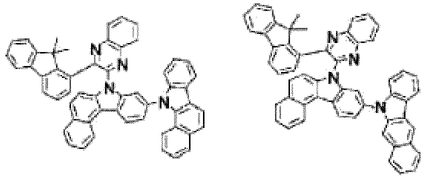

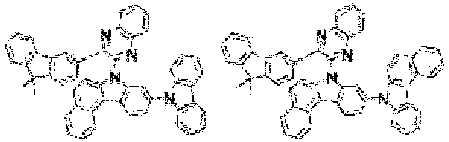

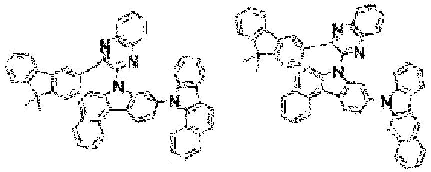

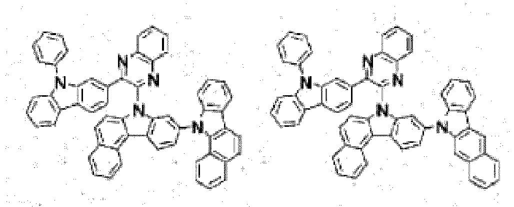

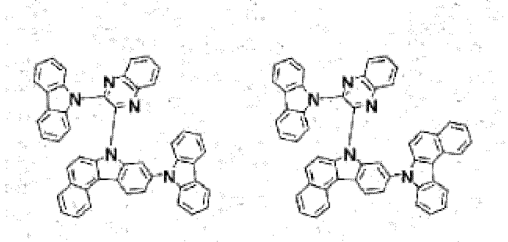

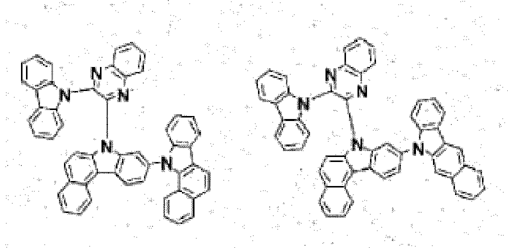

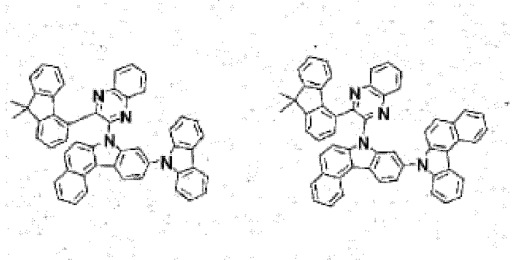

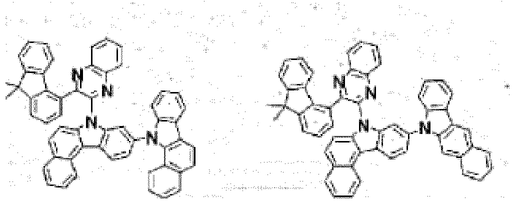

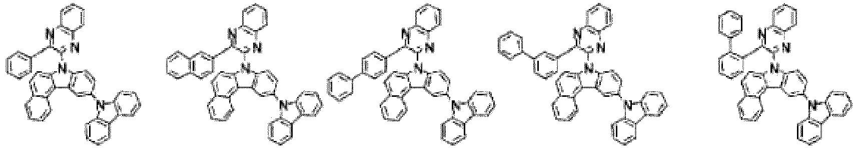

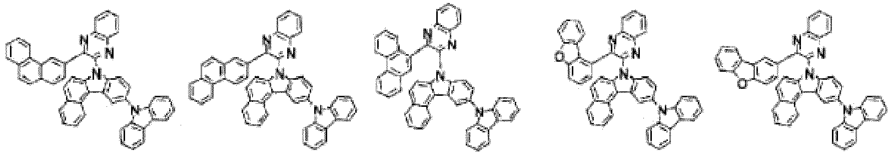

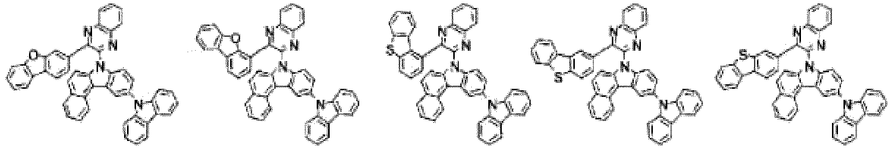

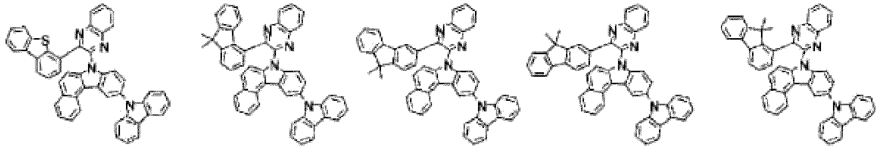

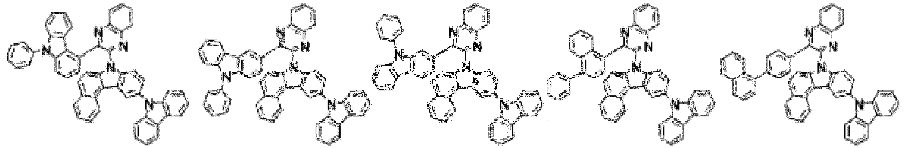

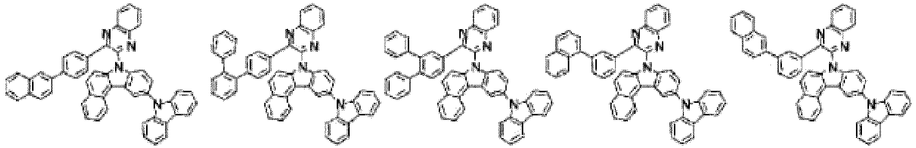

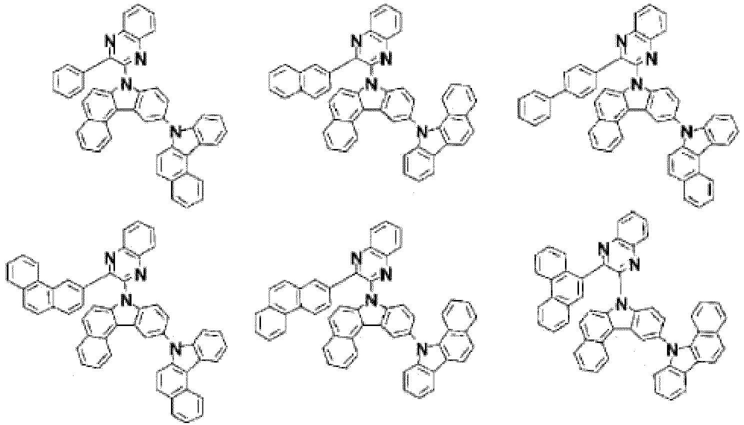

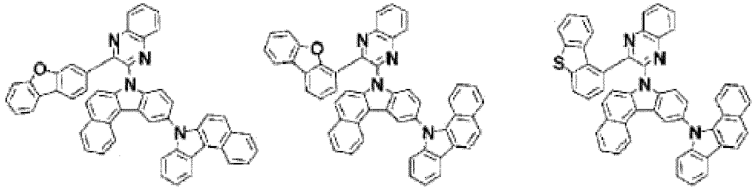

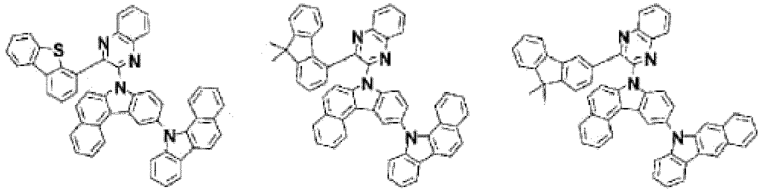

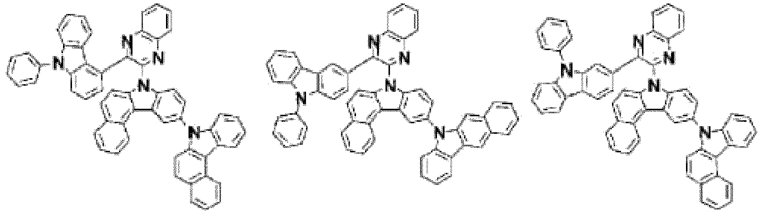

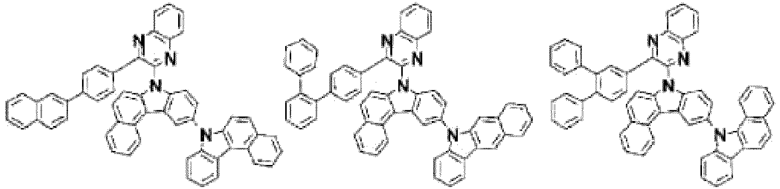

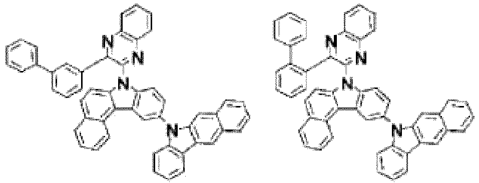

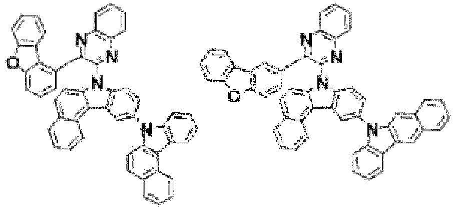

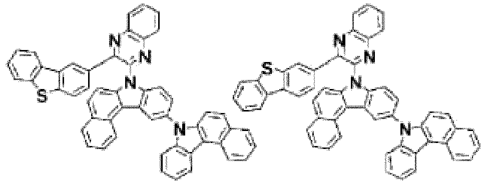

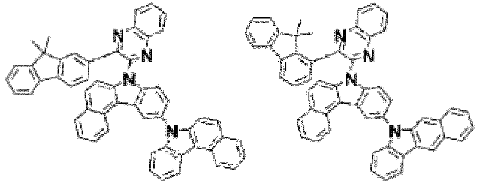

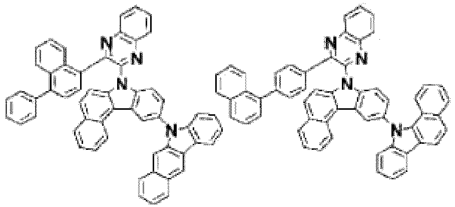

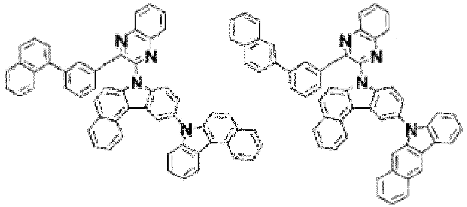

6. Dispositif électroluminescent organique comprenant :
une première électrode ;
une seconde électrode disposée à l'opposé de la première électrode ; et
une, deux ou plus couches de matériau organique disposées entre la première électrode et la seconde électrode,
dans lequel une ou plusieurs couches des couches de matériau organique incluent le composé selon l'une quelconque des revendications 1 à 5.

7. Dispositif électroluminescent organique selon la revendication 6, dans lequel la couche de matériau organique inclut une couche électroluminescente, et la couche électroluminescente inclut le composé.
